# EUROPEAN PATENT APPLICATION

(11) **EP 4 640 208 A1**
(43) Date of publication of application: **29.10.2025**
(21) Application number: 25172442.3
(22) Date of filing: 24.04.2025
(51) Int. Cl.: A61K 8/81, A61Q 5/06, A61Q 17/04

(54) **PERSONAL CARE COMPOSITION**

(30) Priority: 26.04.2024 US 202463639303 P
(71) Applicant: Nouryon Chemicals International B.V., 1101 BZ Amsterdam (NL)
(72) Inventor: THOMAIDES, John Socrates, 1101 BZ Amsterdam (NL); HE, Qiwei, 1101 BZ Amsterdam (NL); MORALES, John M., 1101 BZ Amsterdam (NL)
(74) Representative: Ingrassia, Fisher & Lorenz UK Ltd.

(57) **Abstract**

This disclosure provides a personal care composition including a) a solvent; b) water present in an amount of from 0 to about 5 wt% based on a total weight of the composition; and c) a polyvinyl alcohol (PVOH) acetal including the reaction product of i. a copolymer having at least two repeating units wherein a first repeating unit is a vinyl alcohol residue and a second repeating unit is a vinyl acetate residue; and ii. an aldehyde; wherein the PVOH acetal has an acetal content of about 30 mol% or less.

## Description

### TECHNICAL FIELD

The present disclosure relates to a personal care composition comprising a polyvinyl alcohol (PVOH) acetal, and method of synthesizing such PVOH acetals.

### BACKGROUND

Personal care compositions, such as sunscreens and hair fixatives, have conventionally been made using synthetic polymers. These polymers provide good performance in personal care applications thanks to their "film-forming" ability, which allows them to create a thin, flexible layer or film over the surface of hair strands, skin, and the like. When applied as hair fixative, the film can act as a barrier to humidity and other environmental factors, helping to maintain a desired hairstyle. When applied as a sunscreen, this film can create a protective layer on the surface of the skin as a protective barrier, helping to improve water resistance, adherence to the skin, and overall efficacy. Additionally, these synthetic polymers are typically soluble in organic solvents commonly used in personal care applications.

Conventional synthetic polymers generally provide acceptable skin and hair performance and have adequate solubility in solvents but often lack biodegradability. In contrast, biodegradable polymers often provide insufficient hair and skin performance compared to their conventional counterparts. Accordingly, there is a need to find a polymer suitable in personal care composition that meets both performance and sustainability standards.

FR 3007647 discloses a cosmetic composition including a functionalized polyvinyl alcohol (PVOH). PVOH has a polymer backbone that is biodegradable. As demonstrated in FR 3007647, functionalization of the PVOH is necessary to provide performance in cosmetic compositions, generally to impart a glossy appearance and decrease undesirable material transfer of cosmetic products. More specifically, FR 3007647 describes a polyvinyl alcohol functionalized with at least one aromatic derivative, and optionally an aliphatic derivative, as well as a polyvinyl alcohol that is bifunctionalized with either an aldehyde or a boronic acid. A high degree of functionalization is shown to improve the glossiness of the cosmetic composition when compared to non-functionalized examples but makes the composition less biodegradable. Accordingly, there remains an opportunity for improvement.

### BRIEF SUMMARY

This disclosure provides a personal care composition including:
a) a solvent;
b) water present in an amount of from 0 to about 5 w% based on a total weight of the composition; and
c) a polyvinyl alcohol (PVOH) acetal including the reaction product of:
   i. a copolymer having at least two repeating units wherein a first repeating unit is a vinyl alcohol residue and a second repeating unit is a vinyl acetate residue; and
   ii. an aldehyde;
wherein the PVOH acetal has an acetal content of about 30 mol% or less.

This disclosure also provides a method of synthesizing the polyvinyl alcohol (PVOH) acetal, wherein the method includes the steps of:
a) providing the copolymer; and
b) reacting the copolymer with the aldehyde to form the PVOH acetal.

### DETAILED DESCRIPTION

The following detailed description is merely exemplary in nature and is not intended to limit the current composition. Furthermore, there is no intention to be bound by any theory presented in the preceding background or the following detailed description.

Embodiments of the present disclosure are generally directed to polymers, compositions including the same, and methods for forming the same. For the sake of brevity, conventional techniques related to making polymers and such compositions may not be described in detail herein. Moreover, the various tasks and process steps described herein may be incorporated into a more comprehensive procedure or process having additional steps or functionality not described in detail herein. In particular, various steps in the manufacture of the polymers and associated compositions are well-known and so, in the interest of brevity, many conventional steps will only be described briefly herein or will be omitted entirely without providing the well-known process details.

In this disclosure, the terminology "about" can describe values ± 0.1, 0.5, 1, 2, 3, 4, 5, 6, 7, 8, 9, or 10%, in various embodiments. Moreover, it is contemplated that, in various non-limiting embodiments, it is to be appreciated that all numerical values as provided herein, save for the actual examples, are approximate values with endpoints or particular values intended to be read as "about" or "approximately" the value as recited. It is also contemplated that all isomers and chiral options for each compound described herein are hereby expressly contemplated for use herein in various non-limiting embodiments.

It is to be understood that the subscripts of polymers are typically described as average values because the synthesis of polymers typically produces a distribution of various individual molecules.

The polymers and compositions disclosed herein may suitably include, consist of, or consist essentially of the components, elements, and process delineations described herein. The embodiments illustratively disclosed herein suitably may be practiced in the absence of any element which is not specifically disclosed herein.

The disclosure describes a personal care composition that is biodegradable while still maintaining performance. The personal care composition includes:
a) a solvent;
b) water present in an amount of from 0 to about 5 w% based on a total weight of the composition; and
c) a polyvinyl alcohol (PVOH) acetal including the reaction product of:
   i. a copolymer having at least two repeating units wherein a first repeating unit is a vinyl alcohol residue and a second repeating unit is a vinyl acetate residue; and
   ii. an aldehyde;
wherein the PVOH acetal has an acetal content of about 30 mol% or less.

### Solvent:

The personal care composition includes the solvent. The solvent may be any known in the art. For example, the solvent may be an organic solvent that is polar or non-polar, aromatic or aliphatic, branched or linear. Examples of suitable solvents, without limitation, include ethanol, methanol, isopropanol, acetone, and ethyl acetate. A single solvent or a combination of two or more solvents may also be used. The solvent may be present in an amount of about 80 wt% or less, based on a total weight of the personal care composition.

In one embodiment, the solvent is present at about 1 to about 80 wt%, based on the total weight of the personal care composition. In other embodiments, the solvent is present in an amount of from about 5 to about 75 wt%, about 10 to about 70 wt%, about 15 to about 65 wt%, about 20 to about 60 wt%, about 25 to about 55 wt%, about 30 to about 50 wt%, or about 35 to about 45 wt%, based on a total weight of the personal care composition. In still other embodiments, the solvent is present in an amount of from about 1 to about 10 wt%, about 2 to about 9 wt%, about 3 to about 8 wt%, about 4 to about 7 wt%, or about 5 to about 6 wt%, based on a total weight of the personal care composition. In various non-limiting embodiments, all values and ranges of values including and between those set forth above are expressly contemplated for use herein.

### Water:

The solvent may be anhydrous, defined as having about 0 wt% water. Alternatively, water may be present. When the solvent is not anhydrous and water is present, the water content is typically from greater than about 0 wt% and up to about 5 wt%, based on a total weight of the personal care composition. In one embodiment, the personal care composition is free of water. In another embodiment, the water is present in an amount of from greater than about 0 and up to about 4 wt%, about 1 to about 4 wt%, or about 2 to about 3 wt%. In various non-limiting embodiments, all values and ranges of values including and between those set forth above are expressly contemplated for use herein.

### PVOH Acetal:

The personal care composition also includes the PVOH acetal. The terminology "polyvinyl acetal" or "PVOH acetal" refers to a polymer having a pendent alcohol (OH) group and an acetal group.

As used herein, an acetal is defined as a compound that has a R¹-C(OR²)₂ group where each of R¹ and R² is an alkyl or aryl group.

Typically, a general structure of the polyvinyl alcohol (PVOH) acetal is as follows: 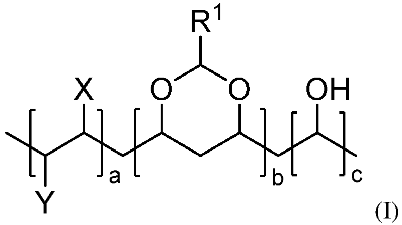
wherein X is or includes a hydrogen or an alkyl or an aryl group that can be linear or branched, saturated or unsaturated, aromatic or non-aromatic;
wherein Y is or includes a hydrogen or an alkyl or an aryl group that can be linear or branched, saturated or unsaturated, aromatic or non-aromatic;
wherein R¹ is an H or a C1-C20 alkyl or aryl group that can be linear or branched, saturated or unsaturated, aromatic or non-aromatic;
wherein a is from about 100 to about 1000;
wherein b is from about 10 to about 1000; and
wherein c is from about 100 to about 1000.

Referring to X and Y, each of X and Y independently is or includes H or an alkyl or an aryl group that can be linear or branched, saturated or unsaturated, aromatic or non-aromatic. In one embodiment, X is CH₃COO- and Y is H, forming a repeating unit that is a vinyl acetate residue. In another embodiment, X is RCOO- where R is a linear or branched alkyl chain. When R is a C11 chain, the repeating unit is a vinyl laurate residue. When R is a C5 chain with an ethyl branch bonded alpha to the COO- group, the repeating unit is a vinyl 2-ethylhexanoate residue. In another embodiment, X is -COOR³ where R³ is a H or an alkyl group, and Y is H, forming a repeating unit that is an acrylic acid residue or an acrylic acid alkyl ester residue where the alkyl group can have a varying chain length. In yet another embodiment, both X and Y are -COOR³, forming a repeating unit that is a maleic acid residue, a maleic acid monoalkyl ester residue, a maleic acid dialkyl ester residue, a fumaric acid residue, a fumaric acid monoalkyl ester residue, or a fumaric acid dialkyl ester residue where the alkyl group can have a varying chain length. In another embodiment, X is -COOR³ and Y is a methyl group, forming a repeating unit that is a crotonic acid residue or a crotonic acid alkyl ester residue where the alkyl group can have a varying chain length. When R³ is H, cyclization with a neighboring vinyl alcohol may or may not occur to form a gamma- and/or a delta-lactone. In one embodiment, X is -COOH and Y is a methyl group, wherein X may or may not cyclize with the neighboring vinyl alcohol to form a crotonic acid gamma-lactone repeating unit.

Multiple different X and Y groups may be present in the same structure and form multiple different repeating units, so long as at least one repeating unit is vinyl acetate. When multiple repeating units comprising different X and Y are present, each can be repeated at different frequencies, also described as having different values for a, so long as the sum of all a values is from about 100 to about 1000.

In one embodiment, a is from about 100 to about 1000. In another embodiment, a is from about 200 to about 900, about 300 to about 800, about 400 to about 700, or about 500 to about 600. In yet another embodiment, a is from about 100 to about 200, about 110 to about 190, about 120 to about 180, about 130 to about 170, or about 140 to about 160. In various non-limiting embodiments, all values and ranges of values including and between those set forth above are expressly contemplated for use herein.

Referring now to R¹, R¹ in structure (I) typically is or includes an H or a C1-C20 alkyl or aryl group, which may be linear or branched, saturated or unsaturated, non-aromatic or aromatic. In one embodiment, R¹ is an H. In another embodiment, R¹ is or includes a C2-C15 alkyl or aryl group, or a C3-C10 alkyl or aryl group. In yet another embodiment, R¹ is a phenyl group. In another embodiment, R¹ is a phenyl group substituted with a sulfonic acid group (-SO₃H) or an alkali metal or alkaline earth metal salt of a sulfonic acid group. Multiple different R¹ groups may be present in the same structure. When multiple R¹ groups are present, each can be repeated at different frequencies, also described as having different values for b, so long as the sum of all b values is from about 10 to about 1000.

In one embodiment, b is from about 10 to about 1000. In another embodiment, b is from about 100 to about 900, about 200 to about 800, about 300 to about 700, about 400 to about 600, or about 500 to about 700. In yet another embodiment, b is from about 10 to about 100, about 20 to about 90, about 30 to about 80, about 40 to about 70, or about 50 to about 60. In various non-limiting embodiments, all values and ranges of values including and between those set forth above are expressly contemplated for use herein.

Now referring to c, in one embodiment, c is from about 100 to about 1000. In another embodiment, c is from about 200 to about 900, or from about 300 to about 800, from about 400 to about 700, or about 500 to about 600. In yet another embodiment, c is from about 100 to about 200, or from about 110 to about 190, or from about 120 to about 180, or from about 130 to about 170, or from about 140 to about 160. In various non-limiting embodiments, all values and ranges of values including and between those set forth above are expressly contemplated for use herein.

The PVOH acetal may alternatively be described as the reaction product of:
a) a copolymer having at least two repeating units wherein a first repeating unit is a vinyl alcohol residue and a second repeating unit is a vinyl acetate residue; and
b) an aldehyde.

### Copolymer:

The copolymer in various embodiments has the general structure: 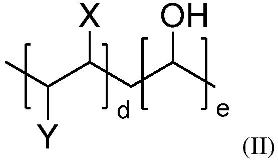
wherein X is or includes a hydrogen or an alkyl or an aryl group that can be linear or branched, saturated or unsaturated, aromatic or non-aromatic;
wherein Y is or includes a hydrogen or an alkyl or an aryl group that can be linear or branched, saturated or unsaturated, aromatic or non-aromatic;
wherein d is from about 100 to about 2000; and
wherein e is from about 100 to about 2000.

Referring to X and Y, X and Y of structure (II) are generally as described in structure (I), and may be the same or different from X and Y in structure (I). Multiple different X and Y groups may be present in the same structure and form multiple different repeating units, so long as at least one repeating unit is vinyl acetate. When multiple repeating units comprising different X and Y are present, each can be repeated at different frequencies, also described as having different values for d, so long as the sum of all d values is from about 100 to about 2000.

In one embodiment, d is from about 100 to about 2000. In another embodiment, d is from about 200 to about 1900, about 300 to about 1800, about 400 to about 1700, about 500 to about 1600, about 600 to about 1500, about 700 to about 1400, about 800 to about 1300, about 900 to about 1200, or about 1000 to about 1100. In yet another embodiment, d is from about 100 to about 200, or from about 110 to about 190, or from about 120 to about 180, or from about 130 to about 170, or from about 140 to about 160. In various non-limiting embodiments, all values and ranges of values including and between those set forth above are expressly contemplated for use herein.

Referring to e, in one embodiment, e is from about 100 to about 2000. In another embodiment, e is from about 200 to about 1900, about 300 to about 1800, about 400 to about 1700, about 500 to about 1600, about 600 to about 1500, about 700 to about 1400, about 600 to about 1300, about 700 to about 1200, about 800 to about 1100, or about 900 to about 1000. In yet another embodiment, e is from about 100 to about 200, about 110 to about 190, about 120 to about 180, about 130 to about 170, or about 140 to about 160. In various non-limiting embodiments, all values and ranges of values including and between those set forth above are expressly contemplated for use herein.

### Aldehyde:

Referring back, the aldehyde is defined as a compound having the structure R⁴CHO, where R⁴ is a H or an alkyl or aryl group, which may be unsubstituted or substituted, branched or linear. In one embodiment, R⁴ is a phenyl group. In another embodiment, R⁴ is a phenyl group substituted with a sulfonic acid group (-SO₃H) or an alkali metal or alkaline earth metal salt of a sulfonic acid group. Examples of aldehydes include, without limitation, benzaldehyde, cinnamaldehyde, 2-formyl benzenesulfonic acid sodium salt, 2-formyl benzenesulfonic acid, 3-formyl benzenesulfonic acid sodium salt, 3-formyl benzenesulfonic acid, 4-formyl benzenesulfonic acid sodium salt, 4-formyl benzenesulfonic acid, 4-hydroxybenzaldehyde, syringaldehyde, ethyl vanillin, ortho-vanillin, isovanillin, salicylaldehyde, ortho-anisaldehyde, para-anisaldehyde, cuminaldehyde, hydroxymethylfurfural, hexyl cinnamaldehyde, jasminaldehyde, 2-formylbenzoic acid, 3-formylbenzoic acid, 4-formylbenzoic acid, glyoxylic acid, formaldehyde, acetaldehyde, propionaldehyde, butyraldehyde, isobutyraldehyde, isovaleraldehyde, vanillin, tolualdehyde, furfural, hexanal, heptanal, octanal, decanal, dodecanal, retinaldehyde, etc. Combinations of two or more aldehydes may also be used.

In one embodiment, the aldehyde is benzaldehyde. In another embodiment, the aldehyde is 2-formyl benzenesulfonic acid or 2-formyl benzenesulfonic acid sodium salt. In yet another embodiment, the aldehyde is a combination of benzaldehyde and 2-formyl benzenesulfonic acid or 2-formyl benzenesulfonic acid sodium salt.

### Physical Properties:

The physical properties of the PVOH acetal are typically governed by the degree of polymerization, the acetal content, and the alcohol content.

The degree of polymerization describes the number of repeating units that are linked together to form a polymer chain. A higher degree of polymerization indicates a longer polymer chain and a heavier molecular weight.

The degree of polymerization of the PVOH acetal is generally indicated by the viscosity-average degree of polymerization derived from the viscosity in water. The degree of polymerization of the PVOH acetal may be from about 500 to about 3000, for example about 500, about 600, about 700, about 800, about 900, about 1000, about 1100, about 1200, about 1300, about 1400, about 1500, about 1600, about 1700, about 1800, about 1900, about 2000, about 2100, about 2200, about 2300, about 2400, about 2500, about 2600, about 2700, about 2800, about 2900, or about 3000. In various embodiments, the degree of polymerization of PVOH acetal is from about 500 to about 2900, about 600 to about 2900, about 600 to about 2800, or about 600 to about 2700, etc. In various non-limiting embodiments, all values and ranges of values including and between those set forth above are expressly contemplated for use herein.

The PVOH acetal can have three or more repeating units wherein a first repeating unit is a vinyl alcohol residue, a second repeating unit is a vinyl acetate residue, and a third repeating unit is a vinyl acetal residue. Additional and optional repeating units may or may not be present in the PVOH acetal. Repeating units in the PVOH acetal can be described by alcohol content, acetate content, and acetal content which refers to the percentage of the vinyl alcohol, vinyl acetate and vinyl acetal repeating unit, based on a total of all the repeating units, indicated in mol%. The total mol% of all repeating units in the PVOH acetal adds up to 100%. The same is true for any polymer and copolymer employed in this disclosure.

Acetal content, also referred to as degree of functionalization, typically describes the proportion of the vinyl acetal repeating unit in all the repeating units, indicated in mol%. In structure (I) when theoretically expressing the acetal content using the repeating units a, b, and c, the acetal content can be expressed as follows: Acetal content (mol%) = (b)/ (a + b + c) × 100. The acetal content can be a useful parameter for determining the biodegradability of the PVOH acetal.

In experimental practice, the acetal content of the PVOH acetal can be quantified using Proton NMR. The acetal content of the PVOH acetal typically is about 30 mol% or less. For example, the acetal content can be about 0 mol%, 1 mol%, about 2 mol%, about 3 mol%, about 4 mol%, about 5 mol%, about 6 mol%, about 7 mol%, about 8 mol%, about 9 mol%, about 10 mol%, about 11 mol%, about 12 mol%, about 13 mol%, about 14 mol%, about 15 mol%, about 16 mol%, about 17 mol%, about 18 mol%, about 19 mol%, about 20 mol%, about 21 mol%, about 22 mol%, about 23 mol%, about 24 mol%, about 25 mol%, about 26 mol%, about 27 mol%, about 28 mol%, about 29 mol%, or about 30 mol%. In other embodiments, the acetal content is from about 0 and up to about 29 mol%, about 1 to about 29 mol%, about 1 to about 28 mol%, etc. In various non-limiting embodiments, all values and ranges of values including and between those set forth above are expressly contemplated for use herein.

Alcohol content typically describes the proportion of the vinyl alcohol repeating unit in all the repeating units, indicated in mol%. In structure (I) when theoretically expressing the alcohol content using the repeating units a, b, and c, the alcohol content can be expressed as follows: Alcohol content (mol%) = (c)/ (a + b + c) × 100. The alcohol content can also be a useful parameter for determining the biodegradability of the PVOH acetal.

In experimental practice, the alcohol content of the PVOH acetal can be quantified using Proton NMR. The alcohol content of the PVOH acetal typically is from about 35 to about 75 mol%. For example, the alcohol content can be about 35 mol%, 36 mol%, about 37 mol%, about 38 mol%, about 39 mol%, about 40 mol%, about 41 mol%, about 42 mol%, about 43 mol%, about 44 mol%, about 45 mol%, about 46 mol%, about 47 mol%, about 48 mol%, about 49 mol%, about 50 mol%, about 51 mol%, about 52 mol%, about 53 mol%, about 54 mol%, about 55 mol%, about 56 mol%, about 57 mol%, about 58 mol%, about 59 mol%, about 60 mol%, about 61 mol%, about 62 mol%, about 63 mol%, about 64 mol%, about 65 mol%, about 66 mol%, about 67 mol%, about 68 mol%, about 69 mol%, about 70 mol%, about 71 mol%, about 72 mol%, about 73 mol%, about 74 mol%, or about 75 mol%. In other embodiments, the alcohol content is from about 35 to about 74 mol%, about 36 to about 74 mol%, about 36 to about 73 mol%, etc. In various non-limiting embodiments, all values and ranges of values including and between those set forth above are expressly contemplated for use herein.

Typically, the personal care composition is also "readily biodegradable," which means that the composition has about 60 wt% biodegradation or more in 28 days or less. The biodegradability of the PVOH acetal may be measured according to OECD 301B. In one embodiment, the PVOH acetal has a biodegradability of greater than about 60 wt%, based on an initial weight of the PVOH acetal, measured after 100 days. In another embodiment, the PVOH acetal has a biodegradability of greater than about 60 wt%, based on an initial weight of the PVOH acetal, measured after 80 days. In yet another embodiment, the PVOH acetal has a biodegradability of greater than about 60 wt%, based on an initial weight of the PVOH acetal, measured after 60 days. In other embodiments, the PVOH acetal has a biodegradability of from about 60 to about 70 wt%, about 60 to about 65 wt%, or about 65 to about 70 wt%, based on an initial weight of the PVOH acetal, measured after 60 days. In various non-limiting embodiments, all values and ranges of values including and between those set forth above are expressly contemplated for use herein.

The PVOH acetal is typically soluble in a solvent e.g. one used in the personal care composition. The solubility of the PVOH acetal in the solvent can be assessed by observing the clarity of the solution made by combining about 10 wt% PVOH acetal solids with about 90 wt% solvent, based on a total weight of the solution. The solution made of PVOH acetal solids in the solvent may or may not be heated to accelerate dissolution. The solvent is typically 100 wt% ethanol or 95 wt% ethanol (balance water). Evaluations are typically made immediately after mixing ("Initial Solubility") and 24 hours later ("24 Hour Solubility") in ambient conditions. The desired result is that the solution made of PVOH acetal solids in the solvent is clear as visually observed in ambient conditions. If the solution is hazy or includes precipitate, this is taken as an indication that the PVOH acetal is at least partly insoluble.

The PVOH acetal may be present in the personal care composition in an amount of from about 1 to about 10 wt%, based on a total weight of the personal care composition. In another embodiment, the PVOH acetal may be present in the personal care composition in an amount of from about 1 to about 9 wt%, or from about 2 to about 8 wt%, or from about 3 to about 7 wt%, or from about 4 to about 6 wt%, based on a total weight of the personal care composition. In another embodiment, the PVOH acetal may be present in the personal care composition in an amount of from about 1 to about 2 wt%, about 1.1 to about 1.9 wt%, about 1.2 to about 1.8 wt%, about 1.3 to about 1.7 wt%, or about 1.4 to about 1.6 wt%, based on a total weight of the personal care composition. In various non-limiting embodiments, all values and ranges of values including and between those set forth above are expressly contemplated for use herein.

The personal care composition may additionally include or be free of by-products resulting from a reaction between the copolymer and an aldehyde, such as water, alkyl acetate, and combinations thereof.

The personal care composition may additionally include or be free of other biodegradable polymers in addition to the PVOH acetal described herein. Such other polymers can be polyglucose polymers including unmodified and modified starches, starch esters, starch ethers, polyesters, polyamides, polyurethanes, etc. and combinations thereof. Suitable additional polymers can be present in an amount of from about 1 to about 99 wt%, based on a total weight of the personal care composition. In one embodiment, one or more additional biodegradable polymers are present in an amount of from about 5 to about 80 wt%, or from about 10 to about 75 wt%, or from about 15 to about 70 wt%, or from about 20 to about 65 wt%, or from about 25 to about 60 wt%, or from about 30 to about 55 wt%, or from about 35 to about 50 wt%, or from about 40 to about 45 wt%, based on a total weight of the personal care composition. In another embodiment, one or more additional biodegradable polymers are present in an amount of from about 5 to about 10 wt%, or from about 6 to about 9 wt%, or from about 7 to about 8 wt%, based on a total weight of the personal care composition. In various non-limiting embodiments, all values and ranges of values including and between those set forth above are expressly contemplated for use herein.

The personal care composition may additionally include or be free of additional non-biodegradable polymers such as: from Nouryon, AMPHOMER^{®} and AMPHOMER^{®} LV-71 polymers (octylacrylamide/acrylates/butylaminoethyl methacrylate com polymer), AMPHOMER^{®} HC^{®} polymer (acrylates/octylacrylamide copolymer), BALANCE^{®} 0/55 and BALANCE CR^{®} polymers (acrylates copolymer), BALANCE^{®} 47 polymer (octylacrylamide/butylaminoethyl methacrylate copolymer), RESYN^{®} 28-2930 polymer (VA/crotonates/vinyl neodecanoate copolymer), RESYN^{®} 28-1310 polymer (VA/Crotonates copolymer), FLEXAN^{®} polymers (sodium polystyrene sulfonate), DynamX polymer (polyurethane-14 (and) AMP-Acrylates copolymer), RESYN XP^{®} polymer (acrylates/octylacrylamide copolymer), STRUCTURE 2001 (acrylates/steareth-20 itaconate copolymer) and STRUCTURE^{®} 3001 (acrylates/ceteth-20 itaconate copolymer); from ISP, OMNIREZ-2000^{®} (PVM/MA half ethyl ester copolymer), GANEX P-904^{®} (butylated PVP), GANEX V-216^{®} (PVP/hexadecene copolymer), GANEX^{®} V-220 (PVP/eicosene copolymer), GANEX^{®} WP-660 (tricontanyl PVP), GANTREZ^{®} A425 (butyl ester of PVM/MA copolymer), GANTREZ^{®} AN-119 PVM/MA copolymer, GANTREZ ES 225^{®} (ethyl ester of PVM/MA copolymer), GANTREZ ES425 (butyl ester of PVM/MA copolymer), GAFFIX VC-713^{®} (vinyl caprolactam/PVP/dimethylaminoethyl methacrylate copolymer), GAFQUAT755^{®} (polyquaternium-11), GAFQUAT HS-100^{®} (poly-quaternium-28), AQUAFLEX XL-306 (Polyimide-1), AQUAFLEX SF-40^{®} (PVP/Vinylcaprolactam/DMAPA Acrylates Copolymer), AQUAFLEX FX-64^{®} (Isobutylene/Ethylmaleimide/Hydroxyethyl maleimide Copolymer), ALLIANZ LT-120^{®} (Acrylates/C1-2 Succinates/Hydroxy acrylates Copolymer), STYLEZE CC-10^{®} (PVP/DMAPA Acrylates Copolymer), STYLEZE 2000^{®} (VP/Acrylates/Lauryl Methacrylate Copolymer), STYLEZE W-20^{®} (Poly quaternium-55), Copolymer Series (PVP/Dimethylamino ethylmethacrylate Copolymer), ADVANTAGE S^{®} and ADVANTAGE LCA^{®} (VinylcaprolactamNP/Dimethylaminoethyl Methacrylate Copolymer), ADVANTAGE PLUS (VA/Butyl Maleate/Isobornyl Acrylate Copolymer), Antaron^{™} ECo (Ethylcellulose); from BASF, ULTRAHOLD STRONG (acrylic acid/ethyl acrylate/t-butyl acrylamide), LUVIMER 100P^{®} (t-butyl acrylate/ethyl acrylate/methacrylic acid), LUVIMER 36D (ethyl acrylate/t-butyl acrylate/methacrylic acid), LUVIQUAT HM-552^{®} (polyquaternium-16), LUVIQUAT HOLD^{®} (polyquaternium-16), LUVISKOL K30 (PVP)LUVISKOL K90^{®} (PVP), LUVISKOL VA 64^{®} (PVP/VA copolymer), LUVISKOL VA73W^{®} (PVPNA copolymer), LUVISKOL VA^{®}, LUVISET PUR^{®} (Polyurethane-1), LUVISET^{®} Clear (VP/Methacrylamide Vinyl Imidazole Copolymer), LUVIFLEX SOFT^{®} (Acrylates Copolymer), ULTRA HOLD 8^{®} (Acrylates/Acrylamide Copolymer), LUVISKOL^{®} Plus (Polyvinylcaprolactam), LUVIFLEX^{®} Silk (PEG/PPG-25/25 Dimethicone/Acrylates Copolymer); from Amerchol, AMERHOLD^{®} DR-25 (acrylic acid/meth acrylic acid/acrylates/methacrylates); from Rohm & Haas, ACUDYNE 258^{®} (acrylic acid/methacrylic acid/acrylates/methacrylates/hydroxy ester acrylates; from Mitsubishi and distributed by Clariant, DIAFORMER Z-301^{®}, DIA FORMER Z-SM^{®}, and DIAFORMER Z-400^{®} (methacryloyl ethyl betaine/acrylates copolymer), ACUDYNE 180^{®} (Acrylates/Hydroxyesters Acrylates Copolymer), ACU DYNE SCP (Ethylenecarboxyamide/AMPSA/Methacrylates Copolymer), and the ACULYN^{®} rheological modifiers; from ONDEO Nalco, FIXOMER A-30^{®} and FIXOMER N-28 ^{®} (INCI names: methacrylic acid/sodium acrylamidomethyl propane sulfonate copolymer); FIXATE G-100^{®} (AMP-Acrylates/Allyl Meth acrylate Copolymer), FIXATE PLUS^{®} (Polyacrylates-X), CARBOPOL^{®} Ultrez 10 (Carbomer), CARBOPOL Ultrez 20^{®} (Acrylates/C10-30 Alkyl Acrylates Copolymer), AVALLTRE AC^{®} series (Acrylates Copolymer), AVALURE UR^{®} series (Polyurethane-2, Polyurethane-4, PPG-17/IPDI/DMPA Copolymer); polyethylene glycol; water-soluble acrylics; water-soluble polyesters; polyacryl amides; polyamines; polyquaternary amines; styrene maleic anhydride (SMA)resin; polyethylene amine; from Covestro, Baycusan^{®} C 2000 (Polyurethane solution in ethanol (INCI- Polyurethane - 64)), Baycusan^{®} eco E 1000 (water-based polyurethane), Baycusan^{®} C 1010 (water-based polyurethane), Baycusan^{®} C 1008 (water-based polyurethane), Baycusan^{®} C 1001 (water-based polyurethane); from Inolex, Inolex Lexfilm Sun (INCI- Polyester-7 (and) Neopentyl Glycol Diheptanoate), Inolex Lexfilm Sun Natural MB (INCI- Capryloyl Glycerin/Sebacic Acid Copolymer), Inolex WetFilm MB (INCI- Trimethylpentanediol/Adipic Acid/Glycerin Crosspolymer), Inolex Lexfilm Spray (INCI- Polyester-10 (and) Propylene Glycol Dibenzoate) and Inolex Lexorez 100 MB (INCI- Adipic Acid/Diglycol Crosspolymer). Other conventional polymers that are polar solvent soluble or that can be made soluble through neutralization with an appropriate base can also be used. In one embodiment, the personal care composition additionally includes or is free of a starch ester polyglucose polymer.

The personal care composition may include or be free of one or more performance enhancers. Non-limiting examples of these performance enhancers include: absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, reducing agents, skin bleaching agents, skin-conditioning agents, skin protectants, solvents, foam boosters, hydrotropes, solubilizing agents, suspending agents, SPF boosters, waterproofing agents, and viscosity modifiers. In one embodiment, the personal care composition optionally further includes a neutralizing agent. Suitable neutralizing agents include both inorganic and organic compounds. Non-limiting examples of inorganic neutralizing agents include sodium or potassium hydroxide. Non-limiting examples of organic neutralizing agents include amines or alkanolamines. Primary, secondary, and tertiary amines or alkanolamines may be used, such as mono-, di-, and tri- long chain fatty amines containing a C4 to C24 hydrocarbon chain, ethoxylates and propoxylates long chain (C4 to C24) fatty amines and mixtures thereof. In one embodiment, the neutralizing agent is chosen from 2-amino-2-methyl-propanol, 2-amino-2-methyl-1, 3-propanediol aminomethylpropanol, di- methyl stearamine, triethanolamine and combinations thereof.

The personal care composition may further include or be free of one or more cosmetic additives. Non limiting examples of cosmetic additives are absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), SPF boosters, waterproofing agents, viscosity increasing agents (aqueous and nonaqueous), and combinations thereof.

The personal care composition can be further defined as a hair fixative or a sunscreen, dependent on additional components present in the composition. Both hair fixatives and sunscreens typically have the physical property of "film-forming" which allows them to create a thin, flexible layer or film over the surface of hair strands, skin, and the like. When applied as hair fixative, the film can act as a barrier to humidity and other environmental factors, helping to maintain a desired hairstyle. When applied as a sunscreen, this film can create a protective layer on the surface of the skin as a protective barrier, helping to improve water resistance, adherence to the skin, and overall efficacy.

### Hair Fixative:

The personal care composition may be described as a hair fixative and may include or be free of an aerosol propellant. The personal care composition may be conventionally stored in a spray can for ease of product application. In hair fixatives, the aerosol propellant is typically combined with the PVOH acetal, the solvent and/or water, and can be sprayed directly from the spray can.

Suitable aerosol propellants include hydrocarbons having 1 to 5 carbon atoms, including but not limited to methane, ethane, propane, isopropane, butane, isobutane, butene, pentane, isopentane, neopentane, pentene, hydrofluorocarbons (HFCs), including but not limited to 1,1-difluoroethane (HP 152a), chlorofluorocarbons (CFCs), hydrofluoro-olefins (HFOs), nitrogen, ethers including but not limited to dimethyl ether, and any combinations thereof. In a typical embodiment, the propellant is dimethyl ether (DME). In one hair fixative embodiment, the aerosol propellant is present in an amount of from greater than about 0 and up to about 90 wt%, based on a total weight of the personal care composition. In various embodiments, the aerosol propellant is present in an amount of from greater than about 0 and up to about 90 wt%, about 5 wt% to about 85 wt%, about 10 wt% to about 80 wt%, about 15 to about 75 wt%, about 20 to about 70 wt%, about 25 to about 65 wt%, about 30 to about 60 wt%, about 35 to about 55 wt%, or from about 40 to about 50 wt%, based on a total weight of the personal care composition. In another embodiment, the aerosol propellant is present in an amount of from about 0 and up to about 10 wt%, about 1 to about 9 wt%, about 2 to about 8 wt%, about 3 to about 7 wt%, or from about 4 to about 6 wt%, based on a total weight of the personal care composition. In various non-limiting embodiments, all values and ranges of values including and between those set forth above are expressly contemplated for use herein.

The hair fixative may further include or be free of additives commonly used in hair care products such as UV absorbers, dyes, perfumes, preservatives, vitamins, moisturizers, anti-itch, anti-dandruff ingredients and combinations thereof.

Hair fixing performance can generally be described through metrics such as high humidity curl retention and other observable on-hair properties such as beading, gloss, stiffness, spring, webbing, feel, shampoo removability. Such metrics are typically evaluated using a Half-Head Wig Evaluation test. Specifically, the hair fixing performance of the composition is compared against a control in a blind evaluation by a panel of judges. Differences in a performance between the composition and the control are regarded as "no significant difference" when chosen two to six times out of eight by the judges, "significantly inferior" when chosen zero or one time, or "significantly superior" when chosen seven or eight times.

Other metrics describing hair fixative performance include viscosity and spray rate. Viscosity can be a factor in the balancing of spray characteristics. Having a low viscosity allows the hair fixative to be sprayed into small droplets and provide uniform coverage. The droplets typically have a volume moment mean D [4,3] as measured using a Spraytec - Open Spray Malvern Particle Size Analyzer, utilizing 300 mm lens, with a particulate refractive index of 1.33 + 0.000i, a dispersant refractive index of 1.00, a particle density of 1.00 gm/cc, a residual of 0.67%, a path length of 100.0 mm, a scatter start value of 6, a scatter end value of 36, and a scattering threshold of 1. The D [4,3] is typically from about 25 to about 150 microns, about 50 to about 125 microns, or about 75 to about 100 microns. In various non-limiting embodiments, all values and ranges of values including and between those set forth above are expressly contemplated for use herein.

The viscosity of the composition is measured using a Brookfield DV-I Prime with a UL Adapter at ambient temperature and a spindle speed of 50 rpm. In one embodiment, the viscosity of a 5 wt% PVOH acetal solution in a solvent system consisting of water and ethylene glycol dimethyl ether at relative weight percent of 67/33 and at 25° C is from about 2 to about 10 cp, about 3 to about 9 cp, about 4 to about 8 cp, or about 5 to about 7 cp. In various non-limiting embodiments, all values and ranges of values including and between those set forth above are expressly contemplated for use herein.

Spray rate in hair fixatives generally refers to the amount of product that is dispensed per unit of time when a spray nozzle is activated. In one embodiment, the hair fixative has a spray rate of from about 0.3 to about 1.5 grams/sec. In another embodiment, the spray rate is from about 0.4 to about 1.4 grams/sec, about 0.5 to about 1.3 grams/sec, about 0.6 to about 1.2 grams/sec, about 0.7 to about 1.1 grams/sec, or about 0.8 to about 1.0 grams/sec. In various non-limiting embodiments, all values and ranges of values including and between those set forth above are expressly contemplated for use herein.

### Sunscreen:

The personal care composition can alternatively be described as a sunscreen and include one or more sunscreen active agents. One skilled in the art may choose an appropriate sunscreen active agent based on the desired performance.

Non-limiting examples of sunscreen active agents include para aminobenzoic acid, avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloyl trioleate, ethyl dihydroxypropyl PABA, glyceryl aminobenzoate, lawsone with dihydroxyacetone, red petrolatum, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, bis-ethylhexyloxyphenol methoxyphenyltriazine, 4-methylbenzylidenecamphor, isopentyl 4-methoxycinnamate, or combinations thereof. In one embodiment, the sunscreen active agent is chosen from avobenzone, homosalate, octisalate, octocrylene, oxybenzone and combinations thereof. In another other embodiment, the sunscreen active agent may further include an inorganic pigment for photoprotection, such as titanium dioxide, zinc oxide, iron oxide, zirconium oxide, cerium oxide, or combinations thereof.

The sunscreen active agent may be present in the personal care composition in an amount of from about 1 to about 25 wt%, based on a total weight of the personal care composition. In one embodiment, the sunscreen active agent is present in an amount of from about 1 to about 25 wt%, about 5 to about 20 wt%, or about 10 to about 15 wt%, based on a total weight of the personal care composition. In another embodiment, the sunscreen active agent is present in an amount of from about 1 to about 10 wt%, about 2 to about 9 wt%, about 3 to about 8 wt%, about 4 to about 7 wt%, about 5 to about 6 wt%, based on a total weight of the personal care composition. In various non-limiting embodiments, all values and ranges of values including and between those set forth above are expressly contemplated for use herein.

The sunscreen may further include or be free of one or more emollients. Emollients can include any appropriate oil, solvent, ester, triglyceride, etc. For suncare products, typical emollients include but not limited to triheptanoin, isopropyl palmitate, C13-C16 isoparaffin, heptyl undecylenate, caprylic/capric triglyceride, diisooctyl succinate, C12-C15 alkyl benzoate, or combinations thereof.

Sunscreens are typically dispensed from bag-on-valve devices. Generally, a bag-on-valve device includes a spray can fitted with an aerosol valve and a welded bag. Unlike the hair fixative, the sunscreen does not typically include the aerosol propellant as an internal component of the composition. Rather, the propellant is typically disposed in a space between the bag and the can of the bag-on-valve device. The sunscreen can be dispensed by the propellant squeezing the bag when a spray button is pressed.

The sunscreen can be applied to the skin as a liquid rub on or as a spray. However, the sunscreen is not limited to skin applications only, and may be extended to usage in other topical regions that may need sun protection. Some non-limiting examples of other topical formulations include lipstick, make-up, lip-balm, eye-shadow, conditioners, and the like.

Sunscreen performance is typically described using sun protection factor (SPF), more specifically with static SPF or water resistance (WR). Static SPF refers to the SPF of the sunscreen when applied to the skin without being exposed to water or other external factors that may affect its efficacy. The static SPF is typically from about 10 to about 80. In one embodiment, the static SPF is from about 10 to about 80, about 15 to about 75, about 20 to about 70, about 25 to about 65, about 30 to about 60, about 35 to about 55, or about 40 to about 50. In various non-limiting embodiments, all values and ranges of values including and between those set forth above are expressly contemplated for use herein.

WR SPF refers to the SPF of the sunscreen when it is water-resistant. Water-resistant sunscreens are formulated to maintain their SPF level when exposed to water or sweat, typically from about 10 to about 80. In one embodiment, the WR SPF is from about 10 to about 80, about 15 to about 75, about 20 to about 70, about 25 to about 65, about 30 to about 60, about 35 to about 55, or about 40 to about 50. In various non-limiting embodiments, all values and ranges of values including and between those set forth above are expressly contemplated for use herein.

### Method of Synthesizing:

The disclosure also provides a method of synthesizing a PVOH acetal, the method includes the steps of:
a) providing a copolymer having at least two repeating units wherein a first repeating unit is a vinyl alcohol residue, and a second repeating unit is a vinyl acetate residue; and
b) reacting the copolymer with an aldehyde to form a PVOH acetal.

The copolymer described in a) may be obtained commercially or may be any described herein. In one embodiment, the copolymer is a 40 mol% hydrolyzed PVOH, obtained from Kuraray Co., Ltd and has a commercial name POVAL^{™} LM-10 HD.

The copolymer described in a) may also be synthesized using techniques well-known in the art. In one embodiment, the copolymer in a) is a reaction product of a polymer having a repeating unit that is a vinyl acetate residue with a primary and/or secondary alcohol. The alcohol partially hydrolyzes the polymer and forms the copolymer described in a).

Referring to the alcohol, a primary alcohol can be defined as a compound having the structure R⁵CH₂OH, where R⁵ is an alkyl or aryl group. A secondary alcohol can be defined as a compound having the structure R⁵R⁶CHOH, where each of R⁵ and R⁶ independently is an alkyl or aryl group. Suitable alcohols, without limitation, include methanol, ethanol, propanol, butanol, pentanol, isopropanol, sec-butanol, 2-pentanol, 2-hexanol, etc., and combinations thereof. Suitable alcohol compounds may have both primary and secondary alcohol groups within the same compound. The alcohol may be the only solvent or may be combined with another non-alcohol solvent. In one embodiment, ethanol acts as both the solvent and the primary alcohol reacting with the polymer. In another embodiment, ethanol is combined with ethyl acetate before reacting with the polymer.

The step of reacting the polymer with the alcohol may be performed at a temperature of from about 40°C to about 100°C, typically from about 60 °C to about 80 °C, at atmospheric pressure and under continuous stirring, during a period of from about 2 to about 12h, in the presence of a catalyst. The catalyst is preferably an acid catalyst. Non-limiting examples of suitable catalysts include para-toluenesulfonic acid, hydrochloric acid, sulfuric acid and combinations thereof. In one embodiment, the catalyst is sulfuric acid.

As described above, the acetal content of the PVOH acetal can be a useful parameter to determine the biodegradability and functionality of the PVOH acetal and the personal care composition. Accordingly, the molar ratio of the copolymer with the aldehyde can be any such that the resulting PVOH acetal has an acetal content of about 30 mol% or less.

As described above, the alcohol content of the PVOH acetal can also be a useful parameter to determine the biodegradability of the PVOH acetal and the personal care composition, in addition to the solubility of the PVOH acetal in the solvent. Accordingly, the molar ratio of the copolymer with the aldehyde is typically considered so that the resulting PVOH acetal has an alcohol content of from about 35 to about 75 mol%.

The step of reacting the copolymer with the aldehyde to form the PVOH acetal may be performed at a temperature of from about 40°C to about 100°C, typically from about 60 °C to about 80 °C, at atmospheric pressure and under continuous stirring, during a period of from about 2 to about 12h, in the presence of a catalyst. The catalyst is preferably an acid catalyst. Non-limiting examples of suitable catalysts include para-toluenesulfonic acid, hydrochloric acid, sulfuric acid and combinations thereof. In one embodiment, the catalyst is sulfuric acid.

The steps of synthesizing the PVOH acetal may be performed sequentially, semi-sequentially or in parallel, dependent on availability of equipment and different manufacturing needs.

In a sequential method, the step of reacting the polymer with the alcohol is typically performed to completion before the step of reacting the copolymer with the aldehyde to form the PVOH acetal commences. Typically, more than about 40 mol% of the vinyl acetate residue is hydrolyzed before the aldehyde is introduced into the reaction. In one embodiment, about 45 mol%, about 50 mol%, about 55 mol %, about 60 mol%, about 65 mol%, about 70 mol%, about 75 mol%, about 80 mol%, about 85 mol%, or about 90 mol% of the vinyl acetate residue is hydrolyzed before the aldehyde is introduced into the reaction. In this embodiment, the aldehyde is only added when its presence is needed to form PVOH acetal. In various non-limiting embodiments, all values and ranges of values including and between those set forth above are expressly contemplated for use herein. This method can be employed to avoid premature degradation of the aldehyde.

In a parallel method, all reactants including the polymer, the alcohol and the aldehyde can be combined at the start of the reaction. This method is convenient and less labor-intensive but presents a drawback of exposing aldehyde to premature degradation.

In a semi-sequential method, the step of reacting the polymer with the alcohol is typically not performed to completion before the aldehyde is introduced. Typically, about 10 to about 40 mol% of the vinyl acetate residue is hydrolyzed before the aldehyde is introduced into the reaction. In one embodiment, about 5 to about 35 mol%, about 10 to about 30 mol%, or about 15 to about 25 mol% of the vinyl acetate residue is hydrolyzed before the aldehyde is introduced into the reaction. In various non-limiting embodiments, all values and ranges of values including and between those set forth above are expressly contemplated for use herein. This method of manufacturing can be employed to balance between lessening the reaction time and avoiding unnecessary degradation of the aldehyde. A person skilled in art may choose a method of manufacturing as described above to suit needs and available equipment.

The method of synthesizing the PVOH acetal typically utilizes an acid catalyst, such as para-toluenesulfonic acid, hydrochloric acid, and sulfuric acid, which is not suitable for personal care applications. Therefore, the catalyst is typically removed from the PVOH acetal before making the personal care composition. Various methods of removing catalysts are known in the art. The catalyst may be neutralized with a suitable base. Suitable bases include but are not limited to alkali metal hydroxide, carbonate, bicarbonate salts, alkaline earth metal hydroxide, carbonate, bicarbonate salts, or combinations thereof. In one embodiment, a basic ion exchange resin is used to remove the acid catalyst.

As known by one skilled in the art, the synthesis of a PVOH acetal typically also form by-products such as water, alkyl acetate, and combinations thereof. One or more by-products may optionally be removed from the PVOH acetal by any number of methods known in the art.

### Additional Embodiments - PVOH Ketals

This disclosure also provides a personal care composition includes:
a) a solvent;
b) water present in an amount of from 0 to about 5 w% based on a total weight of the composition; and
c) a polyvinyl alcohol (PVOH) ketal including the reaction product of:
   i. a copolymer having at least two repeating units wherein a first repeating unit is a vinyl alcohol residue and a second repeating unit is a vinyl acetate residue; and
   ii. a ketone;
wherein the PVOH ketone has a ketal content of about 30 mol% or less; and

The PVOH acetal may be replaced by or used in conjunction with, a PVOH ketal in the personal care composition. Unlike the PVOH acetal, the PVOH ketal includes the reaction product of the copolymer and a ketone. Typically, a general structure of the polyvinyl alcohol (PVOH) ketal is as follows: 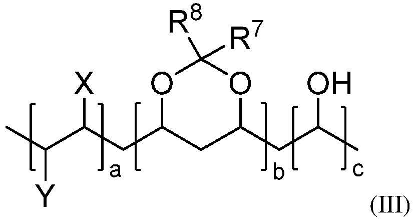
wherein X is or includes a hydrogen or an alkyl or an aryl group that can be linear or branched, saturated or unsaturated, aromatic or non-aromatic;
wherein Y is or includes a hydrogen or an alkyl or an aryl group that can be linear or branched, saturated or unsaturated, aromatic or non-aromatic;
wherein R⁷ is a H or a C1-C20 alkyl or aryl group that can be linear or branched, saturated or unsaturated, aromatic or non-aromatic;
wherein R⁸ is a H or a C1-C20 alkyl or aryl group that can be linear or branched, saturated or unsaturated, aromatic or non-aromatic;
wherein a is from about 100 to about 1000;
wherein b is from about 10 to about 1000; and
wherein c is from about 100 to about 1000;

X, Y, a, b, and c in structure (III) may be the same as or different from X, Y, a, b, and c in structure (I).

Referring to R⁷ and R⁸, each of R⁷ and R⁸ is typically H or a C1-C20 alkyl or aryl group, which may be linear or branched, saturated or unsaturated, non-aromatic or aromatic. In one embodiment, R⁷ and/or R⁸ is a C2-C15 alkyl or aryl group, or a C3-C10 alkyl or aryl group. In another embodiment, R⁷ and/or R⁸ is a phenyl group. In yet another embodiment, R⁷ and R⁸ are connected in a ring system. Multiple different R⁷ and R⁸ may be present in the same structure and form multiple different repeating units. When multiple repeating units comprising different R⁷ and R⁸ are present, each can be repeated at different frequencies, also described as having different values for b, so long as the sum of all b values is from about 10 to about 1000.

A ketone is generally defined as a compound having the structure R⁷COR⁸, where R⁷ and R⁸ independently is an alkyl or aryl group. Suitable ketones may be any ketone known in the art. Non-limiting examples of ketones include acetone, butanone, cyclohexanone, cyclopentanone, cyclobutanone, diethyl ketone, methyl isobutyl ketone, acetophenone, benzophenone, levulinic acid, 3,3,5-trimethylcyclohexanone, dihydrocarvone, raspberry ketone, zingerone, alpha-thujone, beta-thujone, and combinations thereof.

The ketal content of PVOH ketal can be from about 0 to about 30 mol%, especially about 0 mol%, 1 mol%, about 2 mol%, about 3 mol%, about 4 mol%, about 5 mol%, about 6 mol%, about 7 mol%, about 8 mol%, about 9 mol%, about 10 mol%, about 11 mol%, about 12 mol%, about 13 mol%, about 14 mol%, about 15 mol%, about 16 mol%, about 17 mol%, about 18 mol%, about 19 mol%, about 20 mol%, about 21 mol%, about 22 mol%, about 23 mol%, about 24 mol%, about 25 mol%, about 26 mol%, about 27 mol%, about 28 mol%, about 29 mol%, about 30 mol%, or from about 0 to about 29 mol%, about 1 to about 29 mol%, about 1 to about 28 mol%, etc. In various non-limiting embodiments, all values and ranges of values including and between those set forth above are expressly contemplated for use herein.

The PVOH ketal may additionally have a vinyl acetal residue, so long as the sum of the ketal content and the acetal content is from about 0 to about 30 mol%.

A personal care composition including a combination of PVOH acetal and PVOH ketal wherein PVOH acetal has an acetal content of about 30 mol% or less and the PVOH ketone has a ketal content of about 30 mol% or less is also contemplated.

### EXAMPLES

### Synthesis Example 1: Synthesis of PVOH Acetal from POVAL^{™} LM-10 HD

The PVOH acetal was synthesized by modifying POVAL^{™} LM-10 HD, a commercially acquired copolymer, using the following materials and procedure. The resulting PVOH acetal was examined using NMR to experimentally obtain the content of acetate, alcohol and acetal in mol%. The solubility of the PVOH acetal in 95 wt% ethanol (balance water) and the glass transition temperature T_{g} of the PVOH acetal were also analyzed and reported herein.

### Materials:

| | |
|---|---|
| POVAL^{™} LM-10 HD | 30.08 g |
| benzaldehyde | 2.29 g |
| 2-Formyl benzenesulfonic acid, sodium salt | 0.6874 g |
| Concentrated (98 wt%) sulfuric acid | 0.64 g |
| Ethanol, denatured | 120.01 g |
| AMBERLYST A21 ion exchange resin | 7.63 g |

A 4-neck 500 mL round bottom flask was equipped with an overhead mechanical stirrer, nitrogen inlet-topped reflux condenser, thermocouple, and a stopper. To this flask was charged POVAL^{™} LM-10 HD and 120.01 g ethanol. The mixture was heated to about 70 °C using a hot water bath under a positive pressure of nitrogen and held at this temperature until the POVAL^{™} LM-10 HD had fully dissolved (about 45 minutes).

To the resulting solution was then added benzaldehyde, 2-formyl benzenesulfonic acid, sodium salt, and concentrated sulfuric acid in quick succession. The reaction was then held at about 70 ±1 °C for 5 h. At the end of the hold, the reaction was cooled to about <60 °C, and a total of 7.63 g of AMBERLYST A21 ion exchange resin was added to remove the sulfuric acid catalyst. The ion exchange resin was removed by vacuum filtration, and the resin was washed with an excess of ethanol. The filtrate and washings were combined and then dried to a constant weight first at room temperature and then at about 60 °C, yielding 30.07 g of PVOH acetal.

The PVOH acetal was evaluated using Proton NMR and found to include 53 mol% vinyl acetate repeating units, 42 mol% vinyl alcohol monomer repeating units, 4.8 mol% benzaldehyde acetal repeating units, and 0.7 mol% 2-formyl benzenesulfonic acid, sodium salt acetal repeating units. The PVOH acetal produced a very hazy solution in 100% ethanol. The PVOH acetal dissolved in 95 wt% ethanol (balance water) at a concentration of about 10 wt%. The PVOH acetal readily dispersed in water to give a hazy dispersion. The residual acidity of the copolymer product was measured by titration with 0.1 N NaOH and found to be 0.044 mEq/g. The Tg of the PVOH acetal was 76.5 °C.

### Synthesis Example 2A and 2B: Synthesis of PVOH Acetal from Polyvinyl Acetate

The PVOH acetal was synthesized with polyvinyl acetate as a starting material. The procedure included two steps as described below. The resulting PVOH acetal was examined using NMR to experimentally obtain the content of acetate, alcohol and acetal in mol%. The solubility of the PVOH acetal in 95 wt% ethanol (balance water) and the glass transition temperature T_{g} of the PVOH acetal were also analyzed and reported herein.

### Step A. Preparation of a 35.5 wt% solution of polyvinyl acetate.

### Materials:

| | |
|---|---|
| Polyvinyl acetate Mw: 100,000 | 100.00 g |
| Ethanol, denatured | 145.61 g |
| Ethyl acetate | 36.40 g |

Ethanol and ethyl acetate were charged to 1-pint jar equipped with a magnetic stir bar. To the solvent mixture was added polyvinyl acetate with stirring. The jar was capped and stirring was continued until the polyvinyl acetate had dissolved.

### Step B. Preparation of acetal modified partially hydrolyzed polyvinyl alcohol

### Materials:

| | |
|---|---|
| Polyvinyl acetal Mw 100,000 Da, | 98.70 g, as is basis; |
| 35.5 wt% in ethanol / ethyl acetate | 35.04 g, dry basis |
| Concentrated (98 wt%) sulfuric acid | 0.68 g |
| Ethanol, denatured | 5.77 g |
| benzaldehyde | 2.57 g |
| 2-Formyl benzenesulfonic acid, sodium salt | 0.76 g |
| AMBERLYST A21 ion exchange resin - Example 2A | 3.8 g |
| AMBERLYST A21 ion exchange resin - Example 2B | 3.7 g |

A three neck 250 mL flask was equipped with a stopper, reflux condenser, and thermocouple. To the flask was charged 98.7 g of the polyvinyl acetate solution prepared in Step A. The polymer solution was then heated to reflux using a thermostat-controlled heating mantle. To the refluxing polymer solution was added a solution of 0.68 g concentrated sulfuric acid in 5.77 g ethanol via syringe. The reaction was then held at gentle reflux for 4 hours.

After 4 hours at reflux, a 3.1 g sample was withdrawn from the reaction and reserved for analysis. Immediately thereafter, benzaldehyde and 2-formyl benzenesulfonic acid, sodium salt were added and gentle reflux was continued. Four hours after the addition of benzaldehyde and 2-formyl benzenesulfonic acid, 51.4 g of the reaction was withdrawn from the reaction vessel, and the reaction vessel was cooled and allowed to stand sealed overnight at about 0 °C.

To the 51.4 g sample withdrawn from the reaction vessel was added about 20 g of 95 wt% ethanol (balance water) and 3.8 g AMBERLYST A21 ion exchange resin after cooling to < 50 °C. This mixture was allowed to stir at 22-25 °C for 30 minutes and then the resin was removed by vacuum filtration and washed with ethanol. The filtrate and washes thus obtained were combined and dried to a constant weight first at ambient laboratory temperature and then at 60 °C in a forced air oven. The PVOH acetal obtained in this manner is Synthesis Example 2A. The yield of the PVOH acetal was 13.75 g.

The PVOH acetal of Example 2A was evaluated using by Proton NMR and found to include 51.0 mol% vinyl acetate repeating units, 42.3 mol% vinyl alcohol monomer repeating units, 5.8 mol% benzaldehyde acetal repeating units, and 0.9 mol% 2-formyl benzenesulfonic acid, sodium salt acetal repeating units. The PVOH acetal of Example 2A was soluble in 100 wt% ethanol and 95 wt% ethanol (balance water) at a concentration of about 8 wt%. The PVOH acetal of Example 2A product readily dispersed in water to give a hazy dispersion. The residual acidity of the copolymer product was measured by titration with 0.1 N NaOH and found to be 0.112 mEq/g. The T_{g} of the PVOH acetal of Example 2A was 66.9 °C.

The next day, the reaction mixture was brought back up to mild reflux and held there for an additional 3 hours (total reaction time: 11 hours; reaction time with benzaldehyde and 2-formyl benzenesulfonic acid, sodium salt: 7 hours). The reaction was allowed to cool to < 50 °C, and about 20 g 95 wt% ethanol (balance water) and 3.7 g AMBERLYST A21 ion exchange resin was added. The resulting mixture was stirred at 22-25 °C for 30 minutes and then the resin was removed by vacuum filtration and washed with ethanol. The filtrate and washings thus obtained were dried to a constant weight first at ambient laboratory temperature and then at 60 °C in a forced air oven. The material obtained in this manner is Synthesis Example 2B. The yield of PVOH acetal was 12.0 g.

The PVOH acetal of Example 2B was evaluated using Proton NMR and found to include 31.8 mol% vinyl acetate repeating units, 61.9 mol% vinyl alcohol monomer repeating units, 5.4 mol% benzaldehyde acetal repeating units, and 0.8 mol% 2-formyl benzenesulfonic acid, sodium salt acetal repeating units. The PVOH acetal of Example 2B was soluble in 95 wt% ethanol (balance water) at a concentration of about 8 wt%. The PVOH acetal of Example 2B product readily dispersed in water to give a hazy dispersion. The residual acidity of the copolymer product was measured by titration with 0.1 N NaOH and found to be 0.105 mEq/g. The T_{g} of the PVOH acetal of Example 2B was 100.3 °C.

### Synthesis Example 3 to 5: Additional Syntheses of PVOH Acetals from POVAL^{™} LM-10 HD

Additional PVOH acetals were prepared according to the method of Example 1 using different aldehydes and/or combinations of aldehydes with the exception that in Example 5 the reaction was held at about 70 ±1 °C for about 5.25 h after the addition of the aldehydes. The compositions of these PVOH acetals as determined by NMR are given in Table 1 below.

**TABLE 1. Compositions of Examples 3 to 5 as determined by NMR.**

| | Vinyl acetate repeating units (mol%) | Vinyl alcohol repeating units (mol%) | Benzaldehyde acetal repeating units (mol%) | Cinnamaldehyde acetal repeating units (mol%) | Formyl Benzenesulfonic acid, sodium salt acetal repeating units (mol%) | T_{g} (°C) |
|---|---|---|---|---|---|---|
| Example 3 | 63.4 | 31 | 5.7 | --- | --- | 53.8 |
| Example 4 | 51.4 | 44.9 | --- | 3.8 | --- | 65.1 |
| Example 5 | 54.1 | 41.3 | --- | 3.8 | 0.8 | 61 |

### Synthesis Example 6 to 8: Additional Syntheses of PVOH Acetals from Polyvinyl Acetate

Additional PVOH acetals were prepared according to the method of Example 2A using different aldehydes and/or combinations of aldehydes with the following exceptions: (1) the total reaction time for Example 8 was about 4 h 18 minutes and (2) the total reaction time for Example 9 was about 8 h 36 minutes. The compositions of these PVOH acetals as determined by NMR are given in Table 2 below.

**TABLE 2. Compositions of Examples 6 to 9 as determined by NMR.**

| | Vinyl acetate repeating units (mol%) | Vinyl alcohol repeating units (mol%) | Benzaldehyde acetal repeating units (mol%) | Formyl Benzenesulfonic acid, sodium salt acetal repeating units (mol%) | T_{g} (°C) |
|---|---|---|---|---|---|
| Example 6 | 46.2 | 48 | 5.8 | --- | 75.6 |
| Example 7 | 53.9 | 38.9 | 6.3 | 0.9 | 73.1 |
| Example 8 | 51.5 | 40 | 7.3 | 1.2 | 74.2 |

### Synthesis Example 9: Benzaldehyde Acetal Modification of Polyvinyl Acetate-Co-Crotonic Acid Polymer

PVOH acetal was synthesized from a copolymer having three repeating units including a vinyl alcohol residue, a vinyl acetate residue, and a crotonic acid residue, performed in three separate steps, as described in the following procedure.

### Step 1. Preparation of polyvinyl acetate-co-crotonic acid polymer

### Initial Charge:

| | |
|---|---|
| Crotonic acid | 6.63 g |
| Vinyl acetate | 125.88 g |
| tert-Butyl peroctoate | 5.14 |

### Monomer Feed:

| | |
|---|---|
| Crotonic acid | 19.88 g |
| Vinyl acetate | 377.63 |

### Monomer Flush:

| | |
|---|---|
| Ethyl acetate | 8.82 g |

### Initiator Feed #1:

| | |
|---|---|
| Ethyl acetate | 174.37 g |
| tert-Butyl peroctoate | 5.14 g |

### Initiator Feed #2:

| | |
|---|---|
| Ethanol | 13.25 g |
| tert-Butyl peroctoate | 1.59 g |

### Diluent:

| | |
|---|---|
| Ethanol | 301.04 g |

A four-neck 2L round bottom flask was equipped with a mechanical stirrer; a Claisen adapter fitted with a temperature probe and a reflux condenser; a 500 mL addition funnel (not pressure equalized); and a 125 mL addition funnel (not pressure equalized). To the flask was charged 6.63 g crotonic acid, 125.88 g vinyl acetate, 164.30 g ethyl acetate, and 5.14 g 98 wt% tert-butyl peroctoate (Initial charge). To the 500 mL addition funnel was charged a mixture of 19.88 g crotonic acid and 377.63 g vinyl acetate (Monomer Feed). To the 125 mL addition funnel was charged a mixture of 5.14 g 98 wt% tert-butyl peroctoate 174.37 g ethyl acetate (Initiator Feed #1).

The reaction mixture was brought to reflux with stirring using a hot water bath. After the reaction mixture was at reflux for about 5 minutes, the uniform addition of the Monomer Feed to the reaction mixture over about 4 h was started. When the addition of the Monomer feed was complete, the addition funnel was flushed into the reactor with 8.82 g ethyl acetate (Monomer Flush). After the reaction mixture was at reflux for 1h., the uniform addition of Initiator Feed #1 to the reaction over about 5 h was started. After the addition of Initiator Feed #1 was complete, the reaction was held at reflux for an additional 2 h (total reaction time to this point was about 8 h 5 minutes). The reaction was then cooled and allowed to stand overnight.

After standing overnight, the reaction was brought back to reflux, and a solution of 1.59 g tert-butyl peroctoate in 13.25 g ethanol was added in a single shot (Initiator Feed #2). Heating at reflux was continued for 2 h. after the shot-wise addition of the Initiator Feed #2. The reaction was then allowed to cool below reflux, and 301.04 g ethanol (Diluent) was added. The reaction was stirred until a uniform polymer solution was obtained, and then it was allowed to cool. A clear, colorless viscous polymer solution was obtained with a 44.58 wt% solids in ethanol/ethyl acetate, measured with gravimetric analysis.

### Step 2. Partial hydrolysis of polyvinyl acetate-co-crotonic acid polymer

| | |
|---|---|
| Polymer from Step 1 | 580 g, as is basis; |
| | 258.56 g, 100 wt% solids basis. |
| Ethanol | 258.7 g |
| Sulfuric acid (98 wt%) | 4.807 g |

A four-neck 2L round bottom flask was equipped with a mechanical stirrer; a reflux condenser-topped Barrett trap, temperature probe, and stopper. To the flask were charged 580 g (258.56 g, 100 wt% solids basis) of the polyvinyl acetate-co-crotonic acid polymer solution from Step 1 and 158.7 g ethanol. The resulting mixture was heated to mild reflux using a thermostat-controlled heating mantle. When reflux was achieved a solution of 4.807 g concentrated (98 wt%) sulfuric acid in 10 g ethanol was charged to the reactor. This solution was quantitatively rinsed into the reactor with 40 g ethanol.

The temperature was increased to the point at which distillate could be collected in the Barrett trap. Distillation was continued until a total of 50.2 g ethanol was collected. At this point the Barrett trap was removed leaving just the condenser in place and mild reflux was continued. After about 8 h at reflux, a 360 g sample was removed from the reactor, and the reaction was cooled and allowed to stand sealed overnight at about 0 °C.

The next day, the reaction mixture was carefully brought back up to mild reflux and held there for an additional 2 hours (total reaction time: 10 hours). The reaction was allowed to cool to < 50 °C, and 26 g AMBERLYST A21 ion exchange resin was added. The resulting mixture was stirred at 22-25 °C for about 30 minutes and then the resin was removed by vacuum filtration and washed with ethanol. The yield of filtrate and washings thus obtained was 376 g; with 21.88 wt% solids in ethanol / ethyl acetate.

A small portion of the copolymer solution was dried to a constant weight at 60 °C in a forced air oven. The solid copolymer was evaluated using Carbon-13 NMR and found to comprise 26 mol% vinyl acetate repeating units, 69 mol% vinyl alcohol repeating units, and 5 mol% crotonic acid gamma-lactone repeating units (formed by condensation of crotonic acid repeating units with neighboring vinyl alcohol repeating units).

### Step 3. Preparation of benzaldehyde acetal modified vinyl alcohol copolymer.

| | |
|---|---|
| Copolymer from Step 2 | 121.12 g, as is basis; |
| | 26.5 g, 100 wt% solids basis |
| Benzaldehyde | 1.2587 g |
| Sulfuric acid (98 wt%) | 0.913 g |
| Ethanol | 15.00 g |
| AMBERLYST A21 ion exchange resin | 9.8 g |

A 500 mL 4-neck round bottom flask was equipped with an overhead mechanical stirrer, Claisen adapter fit with a temperature probe and a nitrogen inlet / outlet-topped reflux condenser, a nitrogen purge tube, and a stopper. To the flask was charged 121.12 g of the copolymer solution from Step 2 and 1.2587 g benzaldehyde. The solution was then heated to 67 ± 2 °C with a water bath while purging with super-surface nitrogen. Once the reaction was at 67 ± 2 °C, 0.913 g concentrated sulfuric acid (98 wt%) was added. This was quantitatively rinsed into the reaction with 15 g of ethanol. After 2 h reaction time at 67 ± 2 °C, the reaction was cooled to <50 °C, and 9.8 g of AMBERLYST A21 ion exchange resin was added. The resulting suspension was stirred for 0.5 h and then the ion exchange resin was removed by vacuum filtration. The resin was washed with an excess of ethanol, and the filtrate and washings were combined and then dried to a constant weight first at ambient temperature and then at 60 °C, yielding 27.3 g of PVOH acetal. The solid PVOH acetal was evaluated using Proton NMR and found to comprise 39.6 mol% vinyl acetate repeating units, 52.5 mol% vinyl alcohol repeating units, 5.1 mol% crotonic acid gamma-lactone repeating units (formed by condensation of crotonic acid repeating units with neighboring vinyl alcohol repeating units), and 2.7 mol% benzaldehyde acetal repeating units (formed by reaction of benzaldehyde with adjacent vinyl alcohol repeating units). The Tg of the PVOH acetal was measured and found to be 67.4 °C.

### Synthesis Example 10: Additional Benzaldehyde Acetal Modification of Polyvinyl Acetate-Co-Crotonic Acid Polymer

Another PVOH acetal with a higher level of benzaldehyde acetal repeating units was prepared according to the method of Example 9, Step 3 starting from the copolymer of Example 9, Step 2. The solid PVOH acetal was evaluated using Proton NMR and found to comprise 40.4 mol% vinyl acetate repeating units, 49.5 mol% vinyl alcohol monomer repeating units, 5.2 mol% crotonic acid gamma-lactone repeating units (formed by condensation of crotonic acid repeating units with neighboring vinyl alcohol monomer repeating units), and 4.8 mol% benzaldehyde acetal repeating units (formed by reaction of benzaldehyde with adjacent vinyl alcohol repeating units). The Tg of the PVOH acetal was measured and found to be 71.7 °C.

### Example 11: Preparation of Hair Fixatives with the PVOH Acetals of Example 1 and Example 2A

The PVOH acetals described in Examples 1 and 2A were dissolved in 95 wt% ethanol (balance water) solvent and blended with dimethyl ether (DME) as the propellant to make a composition having 3 wt% PVOH acetal, based on a total weight of the composition. To examine the solubility of the PVOH acetals in the solvent when combined with the propellant, the following procedure is performed:
1. Charge all the ethanol to the main mixing vessel.
2. Begin mixing with propeller agitation and adjust the speed of the mixing until there is a vortex pulled 2/3 of the way down the mixing shaft.
3. Slowly add the PVOH acetal by sifting it into the side of the vortex. Allow the PVOH acetal to disperse completely to make a concentrate of 5 wt% PVOH acetal in the ethanol.
4. Fill the concentrate into aerosol containers and charge with the DME propellant at 40 wt%, based on a total weight of the composition.
5. The compositions are observed for clarity, phases, and any precipitation that may have formed initially and over time.

The desired result is a clear solution, which is taken as an indication that the PVOH acetals are soluble in the ethanol when combined with the DME propellant. On the other hand, if the solution is hazy or opaque or includes precipitate, this is taken as an indication that the composition is at least partly insoluble. The compositions are examined 24 h after preparation. **TABLE 3.** Solubility of the PVOH acetals in the ethanol when combined with the DME propellant.

| **Hair Fixative** | **Sample** | **Appearance after 24 hours** |
|---|---|---|
| 1 | Example 1 | Clear |
| 2 | Example 2A | Clear |

The results show that PVOH acetals of Examples 1 and 2A are soluble in the solvent when combined with the propellant and are suitable for use in the hair fixatives.

### Example 12: High Humidity Curl Retention of PVOH Acetals

Hair fixatives formulated from the PVOH acetals of Examples 1 and 2A were prepared as described in Example 11 and compared to a RESYN^{™} 28-2930 anhydrous (Nouryon) hair fixative in a high humidity curl retention test. RESYN^{™} 28-2930 is a commercial film-forming hair fixative that offers cost-effective stiffness with flexibility in high alcohol hair spray formulas. It also provides strong holding power, manageability, gloss, and adhesion to the hair without flaking.

The following procedure was used to evaluate the objective performance of the hair fixatives including the PVOH acetals relative to high humidity curl retention metrics. The high humidity curl retention properties of the hair fixatives were measured. The tests were each conducted at 70 °F and 90% Relative Humidity over a period of 24 hours. The tests were performed on 10" long x 2-gram swatches of European virgin brown hair (nine replicate swatches per sample). Curl retention testing was performed in a humidity chamber set at 70 °F and 90% Relative Humidity for a total of 24 hours. Percentages of curl retention were recorded at time intervals of 15, 30, 60, 90 min, 2, 3, 4, 5, and 24 hrs. The hair fixatives were tested according to the following procedures:
1. Wet hair swatch, comb through to remove tangles and squeeze out excess water (run swatch between thumb and index finger).
2. Apply sample to swatch, gently "work into" swatch and comb through.
3. Roll swatch on 1/2" diameter Teflon mandrel. Remove rolled swatch from mandrel and secure with two hair clips.
4. Place curls on tray and dry in oven overnight.
5. Remove dried curls from oven and let cool to room temperature.
6. Suspend curls, from bound end of swatch, on graduated clear, transparent curl retention boards.
7. Remove clips from curls and gently unwind with glass rod making sure to "break" the curl.
8. Take initial curl length readings before placing boards and curls into environmental chamber (70° F, 90% Relative Humidity).
9. Record curl lengths at the 15, 30, 60, 90 min, 2, 3, 4, 5, and 24 hour time intervals.
10. At the conclusion of test, remove boards and curls from chamber.
11. Clean used hair swatches.
12. Calculate percentage of curl retention and compare between samples.

More specifically, the high humidity curl retention test was performed in a constant temperature and humidity chamber. Curls were rolled on a mandrel and allowed to dry overnight. The curls were then sprayed with solutions of 3 wt% PVOH acetal in 95 wt% ethanol (balance water) and 40 wt% DME and allowed to dry. Then the curls were hung on a board placed in the oven and the percentage of curl loss was tracked over 24 hrs.

The high humidity curl retention properties of the example hair fixatives formulated with PVOH acetals of Example 1 and 2A were measured and compared to the high humidity curl retention properties of the control hair fixative made with RESYN^{™} 28-2930 according to the procedure of Example 11, with the exception that 100 wt% (anhydrous) ethanol was used in the composition. The results are summarized in Table 4.

**TABLE 4. High humidity curl retention compared to Resyn^{™} 28-2930.**

| | | **Curl Retention Time in Humidity (hours)** | | | | | | | | |
|---|---|---|---|---|---|---|---|---|---|---|
| **Sample** | **Hair Fixative** | **0.25** | **0.5** | **1** | **1.5** | **2** | **3** | **4** | **5** | **24** |
| **Example 1** | 1 | = | = | + | + | + | = | = | = | = |
| **Example 2A** | 2 | = | = | + | + | + | + | + | = | = |

The notation "=" describes a similar percentage of curl retention between the example and the control. The notation "+" describes an improved percentage of curl retention of the example over the control.

The data in Table 4 show that, surprisingly and unexpectedly, both of the inventive PVOH acetals exhibit a performance that is significantly superior to Resyn^{™} 28-2930 in high humidity curl retention at some of the shorter time intervals and that both are not statistically different from Resyn^{™} 28-2930 at the 24 hour time interval.

### Example 13: Evaluation of Subjective On-Hair Properties of the PVOH Acetals

The hair fixatives formulated with the PVOH acetals were tested in the Half-Head Wig Evaluation test.

Equipment/Materials:
Head, Sandy, Root turned manikin, #351, Bur Max Co.
Wig stand
Comb: Ace, black, hard rubber, 5" x 1" or equivalent
Example composition - hair fixative to be evaluated
Control composition

Testing Procedure:
1. Place head on wig stand; comb hair to remove any tangles or snarls.
2. Using comb, divide wig into equal sections (left side and right side).
3. Place a divider in middle of head to protect each side from overspray.
4. Spray the example composition 6 inches from head for 10 seconds to one side of head, and 10 seconds of the control composition to the other side of head. Let head dry 45 minutes at room temperature.
5. Spray two heads for each formulation to be tested (randomly alternate sides of example and control compositions between the two heads).
6. Blind evaluations are then performed on each head by four panel members.
7. The panel member then evaluates a series of criteria and selects which side has more of each property, as described below.
8. Differences in a performance property between the two compositions are regarded as "no significant difference" when chosen two to six times out of eight, "significantly inferior" when chosen zero or one out of eight times, or "significantly superior" when chosen seven or eight out of eight times.

Blind Panel Evaluations:
BEADING: Visually examine each side of the head for dried polymer beads. Choose the side which has more beading.
GLOSS: Visually inspect both sides of wig for degree of gloss. Determine which side has more shine/gloss.
STIFFNESS: Gently handle each side of head and "feel" for differences in stiffness. Choose which side is more rigid or stiff.
SPRING: While holding a hair bundle in one hand, gently pull on an edge with the other hand three times only. Look for spring back, and bounce. Select the side that has more elasticity and more Spring.
WEBBING: While holding a hair bundle in both hands, gently pull outward on each of the edges approximately 4 inches. (Do this three times only to avoid damage to the bonds). The more net-like the better the Webbing.
FEEL: Run fingers down each side of the head and determine preference. Choose the side that feels more silky / cleaner feel.
With the exception of beading, it is desirable to have more of the aforementioned properties.

The PVOH acetals of Examples 1 and 2A were combined at 3 wt% PVOH acetal (with 50 wt% of the residual acidity of the polymers neutralized with 2-amino-2-methyl-1-propanol), 40 wt% DME and remainder 95 wt% ethanol (balance water) and compared to AMPHOMER^{®} (Nouryon) and RESYN^{™} 28-2930 controls. AMPHOMER^{®} is a high performing hair fixative polymer that offers market-leading stiffness. Both control hair spray formulations were made at 3 wt% polymer with 100 wt% (anhydrous) ethanol and 40 wt% DME propellant. The results are shown in Tables 5 and 6.

**TABLE 5. Performance of the PVOH acetals of Examples 1 and 2A vs. AMPHOMER^{®} control.**

| | **Beading** | **Gloss** | **Stiffness** | **Spring** | **Webbing** | **Feel** |
|---|---|---|---|---|---|---|
| **Example 1** | 2/8 | 4/8 | 7/8 | 8/8 | 8/8 | 2/8 |
| **Example 2A** | 3/8 | 4/8 | 8/8 | 8/8 | 8/8 | 0/8 |

**TABLE 6. Performance of the PVOH Acetals of Examples 1 and 2A vs. RESYN^{™} 28-2930 control.**

| | **Beading** | **Gloss** | **Stiffness** | **Spring** | **Webbing** | **Feel** |
|---|---|---|---|---|---|---|
| **Example 1** | 3/8 | 4/8 | 7/8 | 8/8 | 8/8 | 0/8 |
| **Example 2A** | 3/8 | 3/8 | 7/8 | 5/8 | 8/8 | 0/8 |

The performance of example compositions formulated with the PVOH acetals in Examples 1 and 2A was either significantly superior to that of the controls or not statistically different from that of the controls in beading, gloss, stiffness, spring, and webbing. These results demonstrate that, compared to the comparative examples, the inventive composition provides superior performance in hair fixing applications.

### Example 14. Biodegradability of PVOH Acetal

The biodegradability of the PVOH acetal in Example 1 was tested using the OECD biodegradability method 301B. The OECD 301 series ratings include (1) "Ultimately Biodegradable," meaning ≥ 60 wt% biodegradable within a 60-day period; (2) "Readily Biodegradable," meaning >60 wt% biodegradation in 28 days or less; (3) "Inherently Biodegradable," meaning 20 wt% to 60 wt% biodegradation in 28 days; and (4) "Non-Biodegradable," meaning less than 20 wt% biodegradation in 28 days.

The results of the OECD 301 test performed on the PVOH acetal in Example 1 are summarized in Table 7.

**TABLE 7. Biodegradability of the PVOH acetal as determined according to OECD 301B.**

| **Day of study** | **Percent Biodegradation** |
|---|---|
| 0 | 0.00 |
| 1 | -0.3 |
| 3 | 0.8 |
| 7 | 2.1 |
| 14 | 15.6 |
| 21 | 26.7 |
| 28 | 37.9 |
| 36 | 51.5 |
| 42 | 60.8 |
| 49 | 64.8 |
| 60 | 67.1 |

The PVOH acetal in Example 1 underwent 37.9 wt% biodegradation in 28 days and 67.1 wt% biodegradation in 60 days.

The inventive compositions perform similarly or better compared to commercial hair fixatives in metrics such as high humidity curl retention, beading, gloss, stiffness, spring, webbing, feel, and shampoo removability. Generally, functionalization of the PVOH backbone improves performance but makes the polymer become less biodegradable. Yet, by controlling the acetal content of the PVOH acetal, the inventive compositions meet the standards for both performance and biodegradability.

### Example 15. Additional Synthesis of PVOH Acetal Using Polyvinyl Acetate

A PVOH acetal was synthesized using polyvinyl acetate as a starting material, using the following procedure. A 4-neck 2-1 round bottom flask was equipped with a mechanical stirrer, nitrogen inlet-topped reflux condenser, thermocouple, and a stopper. To this flask were charged 250 g of polyvinyl acetate, obtained under the tradename VINNAPAS^{®} B 100 SPECIAL (a vinyl acetate homopolymer, CAS no. 9003-20-7), 120 g ethyl acetate and 360 g of SDA-40B ethanol, the latter being added in two fractions to promote polymer solubilization, to form a mixture.

The mixture was treated with 4.79 g of sulfuric acid, allowed to reflux for about 4 hours 5 minutes. Subsequently, 15.0 g of benzaldehyde and 4.476g of 2-formyl benzenesulfonic acid, sodium salt, were added in quick succession and allowed to react for an additional 4 hours 5 minutes. The reaction was cooled to below about 60 °C and 51 g of Amberlyst A21 ion exchange resin, was added to quench the reaction. After 1 h stirring at 60 °C, the reaction was allowed to cool.

The ion exchange resin Amberlyst A21 was removed by vacuum filtration and washed with ethanol, producing a filtrate and washings. The filtrate and washings were combined and dried first under ambient conditions and then in a 60 °C forced air oven, to form dried PVOH acetal. The PVOH acetal was soluble in 95% ethanol (balance water) (tested at an active content of about 5 wt% PVOH acetal). The Tg of the PVOH acetal was 63.4 °C. The PVOH acetal was further analyzed using NMR, and found to include 52 mol% vinyl acetate repeating units, 42 mol% vinyl alcohol repeating units, 4.8 mol% benzaldehyde acetal repeating units, and about 0.6 formyl-Benzenesulfonic acid, sodium salt repeating units.

### Example 16. Benzaldehyde of Example 1 / 2-formyl-Benzenesulfonic acid, sodium salt (OBSA) of example 2

An additional PVOH acetal was synthesized with polyvinyl acetate as a starting material. The procedure included two steps as described below. The resulting PVOH acetal was examined using NMR to experimentally obtain the content of acetate, alcohol and acetal in mol%.

Step A. Preparation of a 33.5 wt% solution of polyvinyl acetate.

### Materials:

| | |
|---|---|
| Polyvinyl acetate Mw: 100,000 | 80.00 g |
| Ethanol, denatured | 111.4 g |
| Ethyl acetate | 37.14 g |

About half of the ethanol and all of the ethyl acetate were charged to 2-1 roundbottom flask. To the solvent mixture was added polyvinyl acetate with stirring. The mixture was stirred at a temperature below 40 °C then heated to about 70 °C. When dissolved, the rest of the ethanol was added.

### Step B. Preparation of acetal modified partially hydrolyzed polyvinyl alcohol

### Materials:

### Product from step 1

| | |
|---|---|
| Concentrated (98 wt%) sulfuric acid | 1.554 g |
| Ethanol, denatured | 6.3 g |
| benzaldehyde | 4.753 g |
| 2-Formyl benzenesulfonic acid, sodium salt | 1.763 g |
| AMBERLYST A21 ion exchange resin | 18 g |

The ½-l flask was equipped with a stopper, reflux condenser, and thermocouple. The polymer solution was then heated to reflux using a thermostat-controlled heating mantle. To the refluxing polymer solution was added a solution of the concentrated sulfuric acid in the additional ethanol. The reaction was then held at gentle reflux for 4 hours.

At this time, benzaldehyde and 2-formyl benzenesulfonic acid, sodium salt were added and gentle reflux was continued. Four hours and ten minutes after the addition of benzaldehyde and 2-formyl benzenesulfonic acid, the reaction vessel was cooled and allowed to stand sealed overnight at about 0 °C.

The mixture was treated with AMBERLYST A21 ion exchange resin and allowed to stir at 22-25 °C for 30 minutes and then the resin was removed by vacuum filtration and washed with ethanol. The filtrate and washes thus obtained were combined, neutralized to a pH of about 6.5, and dried to a constant weight first at ambient laboratory temperature and then at 60 °C in a forced air oven.

This PVOH acetal was evaluated using by Proton NMR and found to include 52.2 mol% vinyl acetate repeating units, 42 mol% vinyl alcohol monomer repeating units, 4.8 mol% benzaldehyde acetal repeating units, and 0.8 mol% 2-formyl benzenesulfonic acid, sodium salt acetal repeating units. The PVOH acetal was soluble in 100 wt% ethanol and dispersible in water at a concentration of about 5 wt%. The T_{g} of the PVOH acetal was 63.8 °C.

### Example 17. Additional Synthesis of a PVOH Acetal with High OBSA Content

A further PVOH acetal was synthesized using the same procedure as Example 15, using 80 g of Vinnapas B100SP polyvinyl acetate. NMR results showed that the PVOH acetal included 54.3 mol% vinyl acetate repeating units, 40.3 mol% vinyl alcohol repeating units, 4.1 mol% benzaldehyde acetal repeating units, and about 1.3 OBSA acetal repeating units.

### Example 18. Synthesis of an Additional PVOH Acetal Using an Ca(OH)₂ Neutralization Method

Another PVOH acetal was synthesized using the following materials and amounts:

| | |
|---|---|
| POVAL^{™} LM-10 HD | 30.02 g |
| benzaldehyde | 2.26 g |
| 2-Formyl benzene sulfonic acid, sodium salt | 0.69 g |
| Concentrated (98 wt%) sulfuric acid | 0.63 g |
| SDA-40B ethanol | 90.39 g |
| Ethyl Acetate | 29.76 g |

A 4-neck 500 mL round bottom flask was set up as described in Example 15. To this flask was charged POVAL^{™} LM-10 HD and ethanol to form a mixture. The mixture was heated using a thermostat-controlled heating mantle under a positive pressure of nitrogen to achieve a temperature of about 50-60 °C, this temperature was maintained for about 45 minutes.

Subsequently, benzaldehyde, 2-Formyl benzene sulfonic acid, sodium salt, ethyl acetate and sulfuric acid were added to the mixture. The mixture was allowed to reflux at about 75 °C for about 4 hours. The reaction was then cooled to less than about 70 °C. An amount of 0.64 g of Ca(OH)₂ was then added to the mixture (rinsed with about 7 g of ethanol) and stirred for about 1 hour. The mixture was allowed to cool to room temperature and let stand overnight.

The mixture was heated to 55 °C with stirring, and a total of 1.14 g Celite 521 filter aid was added. The mixture was then filtered, and the solids washed on the filter with an excess of 95% ethanol (balanced water) to obtain a filtrate and washings. The combined filtrate and washings were then dried to at ambient temperature and then at 60 °C, yielding 30.5 g PVOH acetal.

The PVOH acetal was found to be insoluble in 100 wt% ethanol (tested with an active content of about 10 wt%) but soluble in a 95 wt% ethanol (balance water) (tested with an active content of about 9.6 wt%, appearance clear but slight haziness).

The PVOH acetal was further analyzed using NMR, and found to include 50.8 mol% vinyl acetate repeating units, 43.6 mol% vinyl alcohol repeating units, 4.9 mol% benzaldehyde acetal repeating units, and about 0.7 OBSA acetal repeating units.

### Example 19. Additional High Humidity Curl Retention Results

Hair fixatives formulated with either Example 3 or Example 15 were compared to hair fixatives formulated with POVAL^{™} LM-10 HD or Resyn^{™} 28-2930. The results are summarized in Table 8.

**TABLE 8. Comparison of high humidity curl retention compared to Resyn^{™} 28-2930.**

| | | **Time (hour)** | | | | | | | | | |
|---|---|---|---|---|---|---|---|---|---|---|---|
| **Reference** | **Vs.** | 0 | 0.25 | 0.5 | 1 | 1.5 | 2 | 3 | 4 | 5 | 24 |
| **Resyn^{™} 28-2930** | **POVAL^{™} LM-10 HD** | = | = | = | = | = | = | = | = | = | + |
| | **Example 3** | = | = | = | = | = | = | = | = | + | + |
| | **Example 15** | = | = | = | = | = | = | = | = | = | = |
| **POVAL^{™} LM-10 HD** | **Example 3** | = | = | = | = | = | = | = | = | + | = |
| | **Example 15** | = | = | = | = | = | = | = | = | = | - |
| **Example 3** | **Example 15** | = | = | = | = | = | = | - | - | - | - |

Notations in Table 8 can be interpreted using the description disclosed in Example 12 above. Results in Table 8 show that curl retention performance is approximately the same for all hair fixatives at time point 0 to 2 hours. From time point 4 to 24 hours, hair fixatives formulated using the PVOH acetals produce curl retention results that are either equal to or better than the comparative hair fixatives. More specifically, Examples 3 performs better than both POVAL^{™} LM-10 HD and Resyn^{™} 28-2930 at the 5-hour time point. Examples 3 also outperforms Resyn^{™} 28-2930 at the 24-hour time point. Additionally, Example 15 provide curl retention performance that is equal to the performance of comparative hair fixatives, e.g. at all time points compared to Resyn^{™} 28-2930, at a time point of from about 0 to about 5 hours compared to POVAL^{™} LM-10 HD. These results show that using PVOH acetals to formulate hair fixatives does not compromise hair performance, and in certain examples, performance can be improved.

### Example 20. Formulation and Performance of Sunscreen

The PVOH acetal of Example 15 was utilized to form various sunscreens, using the components as set forth in Table 9.

**TABLE 9. Components and Performance of Sunscreen.**

| **Component** | **Chemical Name** | **SC 15 A** | **SC 15 B** | **SC 15 C** | **SC 15 D** | **SC 15 E** |
|---|---|---|---|---|---|---|
| PVOH acetal (wt%) | Example 15 | 2 | 2 | 2 | 2 | 2 |
| Sunscreen Active Agent (wt%) | Avobenzone | 3 | 3 | 3 | 3 | 3 |
| | Homosalate | 13 | 13 | 13 | 13 | 13 |
| | Octisalate | 5 | 5 | 5 | 5 | 5 |
| | Octocrylene | 9 | 9 | 9 | 9 | 9 |
| Emollient (wt%) | Dicaprylyl ether | --- | 5 | --- | 2.5 | 5 |
| | C12-15 Alkyl Benzoate | 5 | 5 | 5 | --- | --- |
| | Phenylethyl Benzoate | --- | --- | 5 | 2.5 | 5 |
| Solvent (wt%) | Ethanol | 63 | 58 | 58 | 63 | 58 |
| Performance | Static SPF | 33.5 | 44.3 | 51.1 | 40.3 | 46.3 |
| | 80-min WR SPF | 36.1 | 43.3 | 42.1 | 38.5 | 50 |

The sunscreens formed using the PVOH acetal were tested for effectiveness in sun protection, i.e. Sun Protection Factor (SPF), in static condition (static SPF) and when exposed to water, i.e. water-resistant (WR SPF) (also recorded in Table 9).

Specifically, static SPF measures a ratio of the time it takes for sunscreen protected skin to show onset of erythema as compared to a non-sunscreen protected skin. WR SPF measures the aforementioned ratio, after an 80-minute immersion of the skin in water at about 40 °C.

For laboratory measurements of static SPF and WR SPF, a series of in-vitro measurements were made, utilizing artificial skins consisting of partially-hydrophilic acrylic polymer material, Vitro Skin^{™} from Florida Suncare, Inc. A Labsphere UV-1000 Spectrophotometer was used for measurement.

The artificial skin was coated with 20 x 5 mg drops of sunscreen, which were evenly distributed over the 49.6 cm² area of the artificial skin, leading to a coverage of about 2 mg/cm². The UV spectrophotometer was used to measure the absorption of UVA radiation (320-400 nm) and UVB radiation (290-320 nm). Values obtained from the spectrophotometer were used to calculated the SPF.

Results in Table 9 demonstrate that sunscreens can be formulated with the PVOH acetal of Example 15 to achieve satisfactory sun protection. Notably, certain formulations, i.e. SC 15C and SC15E, provide SPF value of above 50, in static condition and when exposed to water, respectively.

### Example 21. Additional Biodegradability

Further biodegradability data was obtained for various PVOH acetals, measured according to OECD 301B, and recorded in Tables 10 and 11 below, in units of wt% of PVOH acetal degraded.

**TABLE 10. Biodegradability of Examples 15, 2B and 19.**

| | **Day** | | | | | | | | |
|---|---|---|---|---|---|---|---|---|---|
| **Sample** | **0** | **4** | **14** | **21** | **28** | **35** | **42** | **49** | **60** |
| **Example 15** | 0 | 1.1 | 12.6 | 29.4 | 51.2 | 58.1 | 61.00 | 63.2 | 65.9 |
| **Example 2B** | 0 | 1.1 | 11.7 | 13.6 | 14.8 | 16.0 | 17.1 | 18.6 | 22.1 |
| **Example 16** | 0 | 1.1 | 21.8 | 32.0 | 47.5 | 56.8 | 60.4 | 62.4 | 64.1 |

**TABLE 11. Biodegradability of Examples 6, 2A, 7, and 8.**

| | **Day** | | | | | | | | | | | |
|---|---|---|---|---|---|---|---|---|---|---|---|---|
| **Sample** | **0** | **7** | **14** | **21** | **28** | **35** | **42** | **49** | **60** | **84** | **100** | **114** |
| **Example 6** | 0 | 0 | 0 | 0 | 1.4 | 4.8 | 11.0 | 16.5 | 22.3 | --- | --- | --- |
| **Example 2A** | 0 | 0 | 6.3 | 11.2 | 14.6 | 21.3 | 32.4 | 42.3 | 50.0 | 56.4 | 58.9 | 60.7 |
| **Example 7** | 0 | 0 | 8.6 | 14.8 | 17.5 | 19.5 | 27.3 | 34.3 | 41.8 | 48.8 | 50.1 | --- |
| **Example 8** | 0 | 0 | 3.6 | 11.2 | 15.0 | 16.3 | 17.5 | 18.3 | 19.0 | --- | --- | --- |

The various PVOH acetal synthesis examples and their biodegradability results show that levels of certain acetals may be customized to improve biodegradability. For example, Example 16 uses the level of benzaldehyde from Example 1 and the level of OBSA from Example 2A/B, but it is distinct from both example 1 and example 2A/B. A number of the other examples increase the levels of both benzaldehyde and OBSA, but in example 16, only the OBSA from example 1 is increased. These Examples shows that without OBSA, there is limited biodegradation (comparing example 1 to example 6) and when comparing Example 1 to example 16, the initial rate of biodegradation further increases.

Additionally, the results above demonstrate that by incorporation acetal modification to PVOH, hair performance can be improved, e.g. high-humidity curl retention, compared to that of an unfunctionalized PVOH. This performance improvement can be achieved without compromising the biodegradability of the PVOH acetal.

The results further show that PVOH acetal may be used in sunscreen, e.g. as a film forming polymer, in sunscreen formulations to form biodegradable sunscreen products, while still providing desirable sun protection performance, i.e. SPF and WR SPF.

The results are surprising because most commercially available PVOHs are not ethanol soluble. The limited number of PVOHs that are ethanol soluble, typically do not provide an acceptable level of performance in intended applications. Additionally, acetal modification of PVOHs can lead to the loss of the biodegradation performance. However, the results described herein demonstrate that certain PVOH acetals and compositions formed therefrom (hair fixatives and sunscreens) can be provide satisfactory performance while being biodegradable.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration in any way. Rather, the foregoing detailed description will provide those skilled in the art with a convenient road map for implementing an exemplary embodiment. It being understood that various changes may be made in the function and arrangement of elements described in an exemplary embodiment without departing from the scope as set forth in the appended claims.

The present disclosure may be further described by the following Aspects.
**Aspect 1.** A personal care composition comprising:
   a) a solvent;
   b) water present in an amount of from about 0 to about 5 wt%, based on a total weight of the composition; and
   c) a polyvinyl alcohol (PVOH) acetal comprising the reaction product of:
      i. a copolymer having at least two repeating units wherein a first repeating unit is a vinyl alcohol residue and a second repeating unit is a vinyl acetate residue; and
      ii. an aldehyde;
   wherein the PVOH acetal has an acetal content of about 30 mol% or less.
**Aspect 2.** The personal care composition of Aspect 1 wherein the PVOH acetal has a biodegradability greater than about 60 wt% measured after 60 days, according to OECD 301B.
**Aspect 3.** The personal care composition of Aspect 1 wherein the PVOH acetal has an alcohol content of from about 35 to about 75 mol%.
**Aspect 4.** The personal care composition of Aspect 1 wherein the copolymer further has one or more repeating units chosen from a vinyl laurate residue, vinyl 2-ethylhaxanoate residue, maleic acid residue, monoalkyl maleate residue, dialkyl maleate residue, fumaric acid residue, monoalkyl fumarate residue, dialkyl fumarate residue, crotonic acid residue, alkyl crotonate residue, acrylic acid residue, acrylic acid ester residue, and combinations thereof.
**Aspect 5.** The personal care composition of Aspect 1 wherein the solvent is chosen from ethanol, methanol, isopropanol, acetone, ethyl acetate, ethylene glycol dimethyl ether, and combinations thereof.
**Aspect 6.** The personal care composition of Aspect 1 wherein the solvent is ethanol.
**Aspect 7.** The personal care composition of Aspect 1 wherein the water is present in an amount from greater than about 0 and up to about 1 wt%, based on a total weight of the composition.
**Aspect 8.** The personal care composition of Aspect 1 wherein the PVOH acetal has an acetal content of about 20 mol% or less.
**Aspect 9.** The personal care composition of Aspect 1 wherein the aldehyde is chosen from benzaldehyde, cinnamaldehyde, 2-formyl benzenesulfonic acid sodium salt, and combinations thereof.
**Aspect 10.** The personal care composition of Aspect 1 wherein the aldehyde has a sulfo group.
**Aspect 11.** The personal care composition of Aspect 1 wherein the copolymer is formed from polyvinyl acetate treated with ethanol.
**Aspect 12.** The personal care composition of Aspect 1 wherein the PVOH acetal has a degree of polymerization of from about 500 to about 3000.
**Aspect 13.** The personal care composition of Aspect 1 wherein the PVOH acetal has a biodegradability greater than about 60 wt% measured after 100 days, according to OECD 301B.
**Aspect 14.** The personal care composition of Aspect 1 wherein the composition is further defined as a hair fixative and further comprises an aerosol propellant.
**Aspect 15.** The personal care composition of Aspect 14 wherein the aerosol propellant is dimethyl ether.
**Aspect 16.** The personal care composition of Aspect 1 wherein the composition is further defined as a sunscreen and further comprises a sunscreen active agent.
**Aspect 17.** The personal care composition of Aspect 16 wherein the sunscreen active agent is chosen from avobenzene, homosalate, octisalate, octocrylene, oxybenzone, and combinations thereof.
**Aspect 18.** A method of synthesizing a polyvinyl alcohol (PVOH) acetal, said method includes the steps of:
   a. providing a copolymer having at least two repeating units wherein a first repeating unit is a vinyl alcohol residue, and a second repeating unit is a vinyl acetate residue; and
   b. reacting the copolymer with an aldehyde to form a PVOH acetal;
   wherein the PVOH acetal has an acetal content of about 30 mol% or less.
**Aspect 19.** The method of Aspect 18 wherein step a) is further defined as reacting a polymer having a repeating unit that is a vinyl acetate residue with a primary and/or secondary alcohol to partially hydrolyze the polymer and form the copolymer.
**Aspect 20.** The method of Aspect 18 wherein step b) occurs in the presence of a catalyst chosen from para-toluenesulfonic acid, hydrochloric acid, sulfuric acid and combinations thereof.

## Claims

1. A personal care composition comprising:
a) a solvent;
b) water present in an amount of from about 0 to about 5 wt% based on a total weight of the composition; and
c) a polyvinyl alcohol (PVOH) acetal comprising the reaction product of:
i. a copolymer having at least two repeating units wherein a first repeating unit is a vinyl alcohol residue and a second repeating unit is a vinyl acetate residue; and
ii. an aldehyde;
wherein the PVOH acetal has an acetal content of about 30 mol% or less.

2. The personal care composition of claim 1 wherein the PVOH acetal has a biodegradability greater than about 60 wt% measured after 60 days, according to OECD 301B.

3. The personal care composition of claim 1 or 2 wherein the PVOH acetal has an alcohol content of from about 35 to about 75 mol%.

4. The personal care composition of any preceding claim wherein the copolymer further has one or more repeating units chosen from a vinyl laurate residue, vinyl 2-ethylhaxanoate residue, maleic acid residue, monoalkyl maleate residue, dialkyl maleate residue, fumaric acid residue, monoalkyl fumarate residue, dialkyl fumarate residue, crotonic acid residue, alkyl crotonate residue, acrylic acid residue, acrylic acid ester residue, and combinations thereof.

5. The personal care composition of any preceding claim wherein the solvent is chosen from ethanol, methanol, isopropanol, acetone, ethyl acetate, ethylene glycol dimethyl ether, and combinations thereof, preferably ethanol.

6. The personal care composition of any preceding claim wherein the water is present in an amount from greater than about 0 and up to about 1 wt%, based on a total weight of the composition.

7. The personal care composition of any preceding claim wherein the PVOH acetal has an acetal content of about 20 mol% or less.

8. The personal care composition of any preceding claim wherein the aldehyde is chosen from benzaldehyde, cinnamaldehyde, 2-formyl benzenesulfonic acid sodium salt, and combinations thereof.

9. The personal care composition of any preceding claim wherein the aldehyde has a sulfo group.

10. The personal care composition of any preceding claim wherein the copolymer is formed from polyvinyl acetate treated with ethanol.

11. The personal care composition of any preceding claim wherein the PVOH acetal has a degree of polymerization of from about 500 to about 3000.

12. The personal care composition of any preceding claim wherein the PVOH acetal has a biodegradability greater than about 60 wt% measured after 100 days, according to OECD 301B.

13. The personal care composition of any preceding claim wherein the composition is further defined as a hair fixative and further comprises an aerosol propellant, wherein the aerosol propellant is preferably dimethyl ether.

14. The personal care composition of any one of claims 1-13 wherein the composition is further defined as a sunscreen and further comprises a sunscreen active agent, wherein the sunscreen active agent is preferably chosen from avobenzene, homosalate, octisalate, octocrylene, oxybenzone, and combinations thereof.

15. A method of synthesizing a polyvinyl alcohol (PVOH) acetal, said method includes the steps of:
a. providing a copolymer having at least two repeating units wherein a first repeating unit is a vinyl alcohol residue, and a second repeating unit is a vinyl acetate residue; and
b. reacting the copolymer with an aldehyde to form a PVOH acetal;
wherein the PVOH acetal has an acetal content of about 30 mol% or less.
